# EUROPÄISCHE PATENTSCHRIFT

(11) **EP 1 991 899 B1**
(45) Veröffentlichungstag und Bekanntmachung des Hinweises auf die Patenterteilung: **11.05.2016**
(21) Anmeldenummer: 07723118.1
(22) Anmeldetag: 08.03.2007
(51) Int. Cl.: G02B 21/22, G02B 26/08, G02B 21/00, G02B 27/64

(54) **MIKROSKOPIESYSTEM MIT EINEM SCHWENKBAREN SPIEGELPAAR ZUR KOMPENSATION EINES OBJEKTVERSATZES**
MICROSCOPY SYSTEM WITH A PIVOTING MIRROR PAIR FOR COMPENSATING AN OBJECT OFFSET
SYSTEME DE MICROSCOPIE COMPRENANT UNE PAIRE DE MIROIRS PIVOTANTS POUR COMPENSER LE DECALAGE D'UN OBJET

(30) Priorität: 08.03.2006 DE 102006010767
(43) Veröffentlichungstag der Anmeldung: 19.11.2008
(73) Patentinhaber: Carl Zeiss Meditec AG, 07745 Jena (DE)
(72) Erfinder: STRÄHLE, Fritz, 73540 Heubach (DE); HAUGER, Christoph, 73431 Aalen (DE)
(74) Vertreter: Diehl & Partner GbR
(86) Internationale Anmeldenummer: PCT/EP2007/002025
(87) Internationale Veröffentlichungsnummer: WO 2007/101695

(56) Entgegenhaltungen:
- EP-A- 1 580 586
- WO-A2-2006/081031
- DE-A1- 10 133 671
- DE-A1- 10 330 581
- GB-A- 2 149 045
- JP-A- 10 260 359
- JP-A- 2002 090 650
- US-A1- 2005 248 837

## Beschreibung

Die vorliegende Erfindung betrifft ein Mikroskopiesystem zur Abbildung eines in einer Objektebene des Mikroskopiesystems anordenbaren Objekts.

Ein vibrationsfestes Mikroskop ist aus der DE 101 33 671 A1 bekannt. Das Mikroskop umfasst ein optisches Mikroskopsystem, das ein Objektbild in einem Feld vorbestimmten Formats erzeugt, einen ersten Sensor zum Messen der Neigung des gesamten Mikroskopsystems, einen zweiten Sensor zum Messen der Bewegung des gesamten Mikroskopsystems, eine Ablenkvorrichtung zum Ablenken von das Mikroskopsystem durchlaufendem Objektlicht in beliebige Richtung und mit beliebigem Winkel, und eine Steuereinheit zum Einstellen der Ablenkrichtung und des Ablenkwinkels des Objektlichts mit der Ablenkvorrichtung abhängig von den Messungen des ersten Sensors und des zweiten Sensors, so dass das Bild trotz einer Neigung oder Bewegung des Mikroskopsystems stationär bleibt.

Das Mikroskopiesystem umfasst wenigstens ein Abbildungssystem, welches wenigstens einen Abbildungsstrahlengang zur Abbildung eines endlichen (d.h. nicht punktförmigen) Abbildungsfeldes der Objektebene bereitstellt, sowie eine Versatzeinrichtung, welche ausgebildet ist, das Abbildungsfeld des Abbildungssystems in der Objektebene translatorisch zu verlagern.

Derartige Mikroskopiesysteme finden beispielsweise in der Medizintechnik als Operationsmikroskope Verwendung, um ein in einer Objektebene angeordnetes Objekt zu beobachten.

Dabei wird das Operationsmikroskop üblicherweise von einem an einer Decke befestigten oder auf dem Boden stehenden Stativ getragen. Das Stativ dient als Versatzeinrichtung, um eine Positionierung des Operationsmikroskops über einem betrachteten Objekt zu erlauben. Hierfür erlaubt das Stativ üblicherweise eine translatorische Verlagerung des Abbildungssystems mit bis zu drei Freiheitsgraden (vor/zurück, links/rechts, oben/unten) sowie zumeist eine rotatorische Bewegung des Operationsmikroskops mit wenigstens einem Freiheitsgrad (Schwenkung gegenüber der Waagrechten).

Derartige Stative sind relativ teuer, da sie einerseits in eingerastetem Zustand eine große Stabilität und Schwingungsfreiheit aufweisen und gleichzeitig leicht betätigbar sein müssen. Hierfür muss das Stativ ausreichend massiv ausgeführt sein, und das von ihm getragene Operationsmikroskop muss durch Federn oder Gegengewichte in möglichst jeder möglichen Stellung des Operationsmikroskops ausbalanciert sein. Weiter muss eine Positionierung mit hoher Genauigkeit möglich sein, da ein Abbildungsfeld (durch ein Abbildungssystem des Operationsmikroskops abgebildeter Bereich der Objektebene) des Operationsmikroskops insbesondere bei hoher Vergrößerung sehr klein ist.

Der vorstehend beschriebene Aufbau eines Mikroskopiesystems bestehend aus einem Operationsmikroskop und einem dieses tragenden Stativ aus dem Stand der Technik weist die folgenden Nachteile auf:
Aufgrund der großen Masse des Operationsmikroskops und des Stativs ist es in Folge der zu beschleunigenden bzw. abzubremsenden trägen Massen auch bei optimal ausbalanciertem Stativ nur schwer möglich, kleinen Bewegungen eines betrachteten Objektes durch Verlagerung des Operationsmikroskops zu folgen oder Feineinstellungen in der Positionierung des Operationsmikroskops vorzunehmen.

Weiter ist die Betätigung eines herkömmlichen Stativs auch bei einer optimalen Ausbalancierung körperlich anstrengend, wenn viele kleine Positionierungen vorgenommen werden müssen. Hierzu trägt die Tatsache bei, dass es eine neue Positionierung des Operationsmikroskops in der Regel erforderlich macht, dass der Benutzer direkt oder mittelbar über eine Steuerung mit seinen Händen tätig wird und somit seine Arbeit unterbrechen muss.

Zur Lösung dieser Probleme ist aus der DE 103 30 581 A1 ein Operationsmikroskopiesystem mit einer mechanisch-motorischen Verlagerungseinrichtung bekannt, welche zwischen dem Operationsmikroskop und dem Stativ eingefügt ist, um das Operationsmikroskop durch einen ansteuerbaren Antrieb relativ zu der Objektebene lateral verlagern zu können, ohne hierbei die Bremsen in den Gelenken des Stativs lösen und die Ausrichtung von Stativglieder relativ zueinander ändern zu müssen.

Diese Verlagerungseinrichtung weist allerdings den Nachteil auf, dass sie technisch aufwendig ist und aufgrund ihres Eigengewichts ein vergleichsweise aufwendigeres und stabileres Stativ notwendig macht. Weiter ist für eine Verlagerung des Operationsmikroskops immer noch eine Beschleunigung der trägen Masse des Operationsmikroskops erforderlich. Die träge Masse des Operationsmikroskops verhindert zum einen eine schnelle Verlagerung. Zum anderen besteht bei einer schnellen positiven oder negativen Beschleunigung der trägen Masse des Operationsmikroskops die Gefahr, dass Schwingungen erzeugt und auf das Stativ übertragen werden. Zudem ist es bei direkter visueller Beobachtung nach einer Verlagerung des Operationsmikroskops erforderlich, dass der Benutzer seine Arbeitsposition zumindest geringfügig anpasst.

Aufgrund der vorstehend beschriebenen Nachteile des Standes der Technik ist es derzeit praktisch nicht möglich, kleine, schnelle und insbesondere periodische Verlagerungen des Operationsmikroskops durchzuführen, um auf beispielsweise durch die Atmung eines Patienten hervorgerufene Verlagerungen eines betrachteten Objektes in der Objektebene zu reagieren.

Das Dokument US 2005/0248837 A1 lehrt das Vorsehen eines in zwei Achsen verschwenkbaren Spiegels zwischen Objektivlinsen eines Mikroskopiesystems, um einen Versatz des Abbildungsfeldes in der Objektebene zu bewirken. Mit der Verschwenkung des Spiegels kann auch die Bewegung eines Manipulators koordiniert werden. Gemäß einer alternativen Lehre wird statt des Verschwenkens einer Spiegelfläche die Verlagerung einer optischen Linse quer zur optischen Achse offenbart.

Das Dokument WO 2006/081031 A2 offenbart ein optisches adaptives Scan-Mikroskop ASOM. Auch hier wird offenbart, dass zwischen dem Objekt und einem verschwenkbaren Spiegel wenigstens eine Objektivlinse angeordnet ist, wobei durch das Verschwenken des Spiegels unterschiedliche Bereiche der Objektebene nacheinander abgetastet werden. Das Gesamtbild wird aus einer Vielzahl derartiger Abtastungen gewonnen. Dieses Dokument wurde erst am 3. August 2006, und somit nach dem Prioritätstag der vorliegenden Patentanmeldung veröffentlicht.

Das Dokument GB 2 149 045 A lehrt, einen Laserstrahl mittels eines Zwei-Achsen-Schwenkspiegels an einer gewünschten Position innerhalb eines Abbildungsfeldes anzuordnen und den Laserstrahl innerhalb des Abbildungsfeldes zu verlagern. Eine Verlagerung des Abbildungsfeldes selber wird nicht offenbart. Das Dokument betrifft insbesondere die Mechanik für ein manuelles Verschwenken des Spiegels.

Ausgehend hiervon ist es eine Aufgabe der vorliegenden Erfindung, ein Mikroskopiesystem bereitzustellen, welches eine Verlagerung eines Abbildungsfeldes der Objektebene auf besonders einfache, schnelle, zuverlässige und vibrationsfreie Weise ermöglicht. Weiter ist es eine Aufgabe der vorliegenden Erfindung, ein Verfahren bereitzustellen, welches eine Verlagerung des Abbildungsfeldes der Objektebene eines Stereo-Mikroskopiesystem auf besonders einfache, schnelle, zuverlässige und vibrationsfreie Weise ermöglicht.

Die vorstehende Aufgabe wird durch ein Mikroskopiesystem mit der Kombination der Merkmale der unabhängigen Ansprüche 1 und 5 gelöst. Vorteilhafte Weiterbildungen finden sich in den Unteransprüchen.

Gemäß einer ersten bevorzugten Ausführungsform umfasst ein Mikroskopiesystem zur Abbildung eines in einer Objektebene des Mikroskopiesystems anordenbaren Objekts ein Abbildungssystem, eine Versatzeinrichtung und eine Steuerung. Das Abbildungssystem stellt wenigstens einen Abbildungsstrahlengang zur Abbildung eines Abbildungsfeldes der Objektebene bereit. Dabei wird unter Abbildungsfeld der endliche (d.h. nicht punktförmige) Bereich der Objektebene verstanden, der zu einem Zeitpunkt von dem wenigstens einen Abbildungsstrahlengang des Abbildungssystems abgebildet werden kann. Das Abbildungsfeld wird im Stand der Technik häufig auch als Objektfeld bezeichnet. Die Versatzeinrichtung ist ausgebildet, das Abbildungsfeld des Abbildungssystems in der Objektebene translatorisch zu verlagern. Die Steuerung ist ausgebildet, einen gewünschten Versatz des Abbildungsfelds in der Objektebene zu ermitteln und die Versatzeinrichtung entsprechend zu steuern. Dabei weist die Versatzeinrichtung eine entlang des wenigstens einen Abbildungsstrahlengangs angeordnete erste Spiegelfläche zur Ablenkung des wenigstens einen Abbildungsstrahlengangs auf, welche erste Spiegelfläche in Abhängigkeit von dem durch die Steuerung ermittelten Versatz verschwenkbar ist.

Somit erfolgt in der ersten Ausführungsform ein gewünschter, durch die Steuerung ermittelter Versatz des Abbildungsfeldes der Objektebene relativ zu der Objektebene durch Verschwenken einer den wenigstens einen Abbildungsstrahlengang des Abbildungssystem ablenkenden ersten Spiegelfläche. Dabei bezeichnet der Begriff "Spiegelfläche" in diesem Dokument allgemein die Reflexionsoberfläche eines optischen Umlenkelements.

Dabei kann die Spiegelfläche beispielsweise frei oder um eine oder mehrere vorgegebene unterschiedliche Schwenkachsen verschwenkbar sein. Die Schwenkachsen können wahlweise außerhalb oder innerhalb der Spiegelfläche liegen. Auch können die Spiegelfläche und die Schwenkachsen wahlweise in der gleichen oder in unterschiedlichen Ebenen liegen. Gemäß einer Ausführungsform sind zwei zueinander orthogonale Schwenkachsen vorgesehen, die sich in einem Bereich der Spiegelfläche schneiden, an dem eine optische Achse eines auf die Spiegelfläche einfallenden Abbildungsstrahlengangs auf die Spiegelfläche trifft. Dieser Bereich kann in der Mitte der Spiegelfläche liegen. Die Schwenkachsen sind gedachte Achsen. Das bedeutet, dass die Spiegelfläche um diese gedachten Achsen herum gedreht wird, und bedeutet nicht, dass eine Welle, wie zum Beispiel ein Stab oder eine Stange, eingebaut sein muss.

Alternativ kann der Versatz des Abbildungsfeldes auch dadurch bewirkt werden, dass die Spiegelfläche um eine Drehachse rotiert wird, die im wesentlichen parallel zu einer von optischen Elementen (insbesondere optischen Linsen) des Abbildungssystems festgelegten optischen Achse des wenigstens einen von dem Abbildungssystem auf die Spiegelfläche einfallenden Abbildungsstrahlenganges ausgerichtet ist. Im Rahmen dieser Anmeldung wird unter einer Drehachse, die im wesentlichen parallel zu der optischen Achse ausgerichtet ist, verstanden, dass sich die jeweils von der Drehachse bzw. der optischen Achse und einer gemeinsamen Normalen aufgespannten Ebenen unter einem Winkel kleiner 20°, vorzugsweise kleiner 5° und besonders bevorzugt kleiner 2° schneiden. Dabei können die Drehachse und die optische Achse auch zusammenfallen. Anders gesagt kann die Spiegelfläche auch um eine Drehachse rotiert werden, die mit einer Normalen zur Spiegelfläche einen von Null verschiedenen Winkel einschließt. Durch eine derartige Rotation der Spiegelfläche wird gleichzeitig ein Verschwenken der Spiegelfläche relativ zu der optischen Achse des wenigstens einen von dem Abbildungssystem auf die Spiegelfläche einfallenden Abbildungsstrahlenganges bewirkt, was den Versatz des Abbildungsfeldes in der Objektebene zur Folge hat.

Ein Verschwenken einer Spiegelfläche ist mit hoher Genauigkeit möglich. Da lediglich die träge Masse eines die Spiegelfläche bereitstellenden Umlenkelements (wie beispielsweise aber nicht abschließend eines optischen Spiegels oder Prismas) sowie eines ggf. vorhandenen Antriebs und/oder Getriebes, nicht jedoch die träge Masse des ganzen Abbildungssystems beschleunigt werden muss, sind auch kleine, schnelle und insbesondere periodische Verlagerungen des Abbildungsfeldes auf besonders einfache und zuverlässige Weise möglich. Dabei arbeitet die Versatzeinrichtung aufgrund der kleinen zu beschleunigenden trägen Masse auch bei starken Beschleunigungen nahezu vibrationsfrei.

Da für den gewünschten Versatz des Abbildungsfeldes lediglich die Spiegelfläche verschwenkt wird, das Abbildungssystem jedoch ortsfest bleibt, kann eine Arbeitsposition eines Benutzers nach dem Versatz des Abbildungsfeldes auch bei direkter visueller Beobachtung unverändert beibehalten werden.

Bei einem Versatz, der beispielsweise weniger als ein Viertel und bevorzugt weniger als ein Achtel eines Arbeitsabstandes des Abbildungssystems von der Objektebene beträgt, erfolgt zudem weiterhin eine nahezu senkrechte Abbildung der Objektebene mittels des Abbildungssystems. Dabei wird jedoch betont, dass auch eine nicht senkrechte Betrachtung der Objektebene durch das Abbildungssystem in den meisten Fällen nicht schädlich ist und sogar gewünscht sein kann.

Gemäß einer Ausführungsform kann die Versatzeinrichtung weiter eine entlang des wenigstens einen Abbildungsstrahlengangs angeordnete zweite Spiegelfläche zur Ablenkung des wenigstens einen Abbildungsstrahlengangs aufweisen, welche zweite Spiegelfläche in Abhängigkeit von dem durch die Steuerung ermittelten Versatz verschwenkbar ist. Dann kann die erste Spiegelfläche um eine erste Schwenkachse und die zweite Spiegelfläche um eine zweite Schwenkachse schwenkbar sein, welche zweite Schwenkachse von der ersten Schwenkachse verschieden ist.

Somit kann die Verlagerung des Abbildungsfeldes auch durch Verschwenken von zwei oder auch mehr Spiegelflächen, welche den wenigstens einen Abbildungsstrahlengang nacheinander ablenken, gemeinsam bewirkt werden. Dies kann Vorteile bringen, da ein Verschwenken einer Spiegelfläche um eine einzige vorgegebene Achse häufig einfacher und mit größerer Genauigkeit möglich ist, als eine freie Verschwenkung einer Spiegelfläche.

Dabei kann die erste Schwenkachse mit einer ersten Ablenkungsebene, die von einer auf die erste Spiegelfläche einfallenden und von der ersten Spiegelfläche ausfallenden optischen Achse des wenigstens einen Abbildungsstrahlengangs aufgespannt ist, einen Winkel von im wesentlichen 90° einschließen. Dabei ist die optische Achse von optischen Elementen (insbesondere optischen Linsen) des Abbildungssystems festgelegt. Weiter kann die zweite Schwenkachse mit einer zweiten Ablenkungsebene, die von der auf die zweite Spiegelfläche einfallenden und von der zweiten Spiegelfläche ausfallenden optischen Achse des wenigstens einen Abbildungsstrahlengangs aufgespannt ist, einen Winkel von im wesentlichen 90° einschließen und gleichzeitig zu der ersten Ablenkungsebene im wesentlichen parallel angeordnet sein.

Dabei wird in dieser Anmeldung unter "im wesentlichen 90°" eine Abweichung von 90° um höchstens 5° und bevorzugt höchstens 2° und besonders bevorzugt höchstens 1° verstanden. Weiter wird in dieser Anmeldung unter einer Schwenkachse, die zu einer Ablenkungsebene im wesentlichen parallel ist, verstanden, dass die Schwenkachse zu der Ablenkungsebene parallel ist oder dass die Schwenkachse die Ablenkungsebene schneidet und dabei mit der Ablenkungsebene einen Winkel von kleiner 5° und bevorzugt kleiner 2° einschließt. Weiter können die Schwenkachse und die Ablenkungsebene alternativ auch zusammenfallen.

Gemäß einer Ausführungsform verlaufen die erste und zweite Schwenkachse jeweils in einem Bereich, in dem die auf die jeweilige erste und zweite Spiegelfläche einfallende optische Achse auf die jeweilige Spiegelfläche trifft. Dieser Bereich kann in der Mitte der jeweils zugehörigen ersten und zweiten Spiegelfläche liegen.

Diese Beziehung der ersten und zweiten Schwenkachse relativ zu dem wenigstens einen Abbildungsstrahlengang bzw. relativ zueinander weist den Vorteil auf, dass ein Verschwenken der ersten und zweiten Spiegelfläche um die Schwenkachsen neben der Verlagerung des Abbildungsfeldes relativ zur Objektebene keine bzw. nur eine vernachlässigbare Rotation der durch das Abbildungssystem erzeugten Abbildung des Abbildungsfelds hervorruft. Weiter wird so eine Translation des Abbildungsfeldes in der Objektebene in zwei Richtungen, die miteinander ebenfalls einen Winkel von im wesentlichen 90° einschließen, ermöglicht.

Alternativ oder zusätzlich kann das Mikroskopiesystem weiter eine Kompensationseinrichtung umfassen, welche eine Rotation der durch das Abbildungssystem erzeugten Abbildung des Abbildungsfelds bewirkt. Dabei steuert die Steuerung die Kompensationseinrichtung in Abhängigkeit von einer Verschwenkung der ersten und/oder zweiten Spiegelfläche so, dass eine durch eine Verschwenkung der Spiegelflächen ggf. hervorgerufene Rotation der durch das Abbildungssystem bereitgestellten Abbildung des Abbildungsfelds durch eine von der Kompensationseinrichtung bewirkte Rotation der Abbildung wieder aufgehoben wird.

Dabei kann das Abbildungssystem wenigstens eine in dem wenigstens einen Abbildungsstrahlengang angeordnete Kamera zur Erzeugung von Bilddaten aufweisen und die Kompensationseinrichtung mit der wenigstens einen Kamera verbunden sein. Dann kann die Kompensationseinrichtung eine Rotation der von der wenigstens einen Kamera erzeugten Bilddaten durch elektronische Bildverarbeitung bewirken, um eine durch eine Verschwenkung der Spiegelflächen ggf. hervorgerufene Rotation der durch das Abbildungssystem bereitgestellten Abbildung des Abbildungsfelds auf besonders einfache Weise zu kompensieren.

Alternativ oder zusätzlich kann die Kompensationseinrichtung wenigstens eine in dem wenigstens einen Abbildungsstrahlengang angeordnete und durch die Steuerung einstellbare Prisma-Anordnung aufweisen. Dabei ist die Prisma-Anordnung bevorzugt ausgebildet, bei einer Verdrehung des Prismas eine Bilddrehung um die von optischen Elementen des Abbildungssystems festgelegte optische Achse als Drehachse zu bewirken.

Gemäß einer zweiten bevorzugten Ausführungsform, die mit der vorangegangenen ersten bevorzugten Ausführungsform auch kombiniert werden kann, umfasst ein Mikroskopiesystem zur Abbildung eines in einer Objektebene des Mikroskopiesystems anordenbaren Objekts ein Abbildungssystem, welches wenigstens einen Abbildungsstrahlengang zur Abbildung eines Abbildungsfeldes der Objektebene bereitstellt, sowie eine Versatzeinrichtung, welche ausgebildet ist, das Abbildungsfeld des Abbildungssystems in der Objektebene translatorisch zu verlagern. Dabei weist die Versatzeinrichtung wenigstens ein Paar von entlang des wenigstens einen Abbildungsstrahlengangs angeordneten ersten und zweiten Spiegelflächen zur Ablenkung des wenigstens einen Abbildungsstrahlengangs auf. Dabei ist das wenigstens eine Paar von Abbildungsstrahlengängen nacheinander an der ersten und der zweiten Spiegelfläche reflektiert. Weiter ist die erste Spiegelfläche um eine erste Schwenkachse verschwenkbar, welche erste Schwenkachse mit einer ersten Ablenkungsebene, die von einer auf die erste Spiegelfläche einfallenden und von der ersten Spiegelfläche ausfallenden optischen Achse des wenigstens einen Abbildungsstrahlengangs aufgespannt ist, einen Winkel von im wesentlichen 90° einschließt. Dabei ist die optische Achse durch optische Elemente (insbesondere optische Linsen) des Abbildungssystems festgelegt. Zudem ist die zweite Spiegelfläche um eine zweite Schwenkachse verschwenkbar, welche zweite Schwenkachse mit einer zweiten Ablenkungsebene, die von der auf die zweite Spiegelfläche einfallenden und von der zweiten Spiegelfläche ausfallenden optischen Achse des wenigstens einen Abbildungsstrahlengangs aufgespannt ist, einen Winkel von im wesentlichen 90° einschließt. Zudem ist die zweite Schwenkachse zu der ersten Ablenkungsebene im wesentlichen parallel angeordnet. Dabei kann die zweite Schwenkachse auch in der ersten Ablenkungsebene liegen.

Durch Verschwenken der ersten und/oder zweiten Spiegelfläche um die erste und/oder zweite Schwenkachse kann so eine translatorische Verlagerung des Abbildungsfeldes in der Objektebene in zwei Richtungen, welche miteinander einen Winkel von im wesentlichen 90° einschließen, bewirkt werden. Dabei führt die genannte Orientierung der ersten und zweiten Schwenkachse relativ zu dem wenigstens einen Abbildungsstrahlengang dazu, dass ein Verschwenken der Spiegelflächen keine bzw. nur eine vernachlässigbare Rotation der durch das Abbildungssystem erzeugten Abbildung des Abbildungsfelds hervorruft.

Wie bei der ersten bevorzugten Ausführungsform kann das Mikroskopiesystem weiter eine Steuerung aufweisen, welche ausgebildet ist, einen gewünschten Versatz des Abbildungsfelds in der Objektebene zu ermitteln und die erste und zweite Spiegelfläche in Abhängigkeit von dem ermittelten Versatz um die jeweilige erste bzw. zweite Schwenkachse zu verschwenken.

Allgemein kann das Abbildungssystem wenigstens eine in dem wenigstens einen Abbildungsstrahlengang angeordnete Kamera zur Erzeugung von Bilddaten aufweisen und die Steuerung mit der wenigstens einen Kamera verbunden und weiter ausgebildet sein, in den Bilddaten automatisch die Position eines Markers zu detektieren und den ersten und/oder zweiten Antrieb automatisch in Abhängigkeit von der detektierten Position des Markers zu steuern.

Dabei kann es sich bei dem Marker beispielsweise um ein separates, in der Objektebene eigens angeordnetes Element (z.B. ein charakteristisch ausgebildeter Aufkleber) oder aber auch um ein in den Bilddaten automatisch identifiziertes charakteristisches Element des Objektes selber (wie z.B. ein in den Bilddaten abgebildetes chirurgisches Element / Instrument oder ein bestimmtes Körperteil eines Patienten wie z.B. ein Zahn oder ein Organ) handeln.

Dabei kann es wünschenswert sein, wenn die Steuerung ausgebildet ist, den ersten und/oder zweiten Antrieb automatisch so zu steuern, dass die Position des Markers in den Bilddaten im wesentlichen konstant bleibt.

Hierdurch ist beispielsweise eine (auch automatische) Bildstabilisierung oder ein (auch automatisches) Nachverfolgen der Bewegung eines betrachteten Objektes möglich. Die Bildstabilisierung ist insbesondere dann wünschenswert, wenn ein betrachtetes Objekt (beispielsweise in Folge von Atmung eines Patienten) periodische Lageveränderungen aufweist. Bei der Nachverfolgung der Bewegung eines Objektes ist es beispielsweise möglich, der Bewegung eines chirurgischen Instrumentes zu folgen und das chirurgische Instrument immer in der Mitte des Abbildungsfeldes zu halten.

Weiter oder alternativ kann die Steuerung auch eine Benutzerschnittstelle wie beispielsweise eine Tastatur, ein Fußpedal, einen Joystick, eine Sprachsteuerung etc. aufweisen, und den gewünschten Versatz des Abbildungsfelds in der Objektebene in Abhängigkeit von einem über die Benutzerschnittstelle empfangenen Steuerbefehl ermitteln.

Dabei kann durch einen Benutzer über die Benutzerschnittstelle beispielsweise ein absoluter Versatz (z.B. in Form von Zielkoordinaten), ein relativer Versatz (z.B. in Form eines von der aktuellen Mitte der Abbildung ausgehenden Versatzvektors) und/oder lediglich eine Richtung eines Versatzes in Verbindung mit einer Zeitspanne (z.B. Halten eines Joysticks in eine gewünschte Richtung bis ein gewünschter Versatz erreicht ist) eingegeben werden.

Dabei kann die Benutzerschnittstelle weiter ausgebildet sein, um Steuerbefehle von einem Benutzer in Form von Sprache und/oder einer Augenbewegung und/oder einer Fußbewegung und/oder einer Kopfbewegung und/oder einer Handbewegung des Benutzers zu empfangen und (in digitaler oder analoger Form) an die Steuerung auszugeben.

Allgemein kann das Abbildungssystem eine Mehrzahl von optischen Linsen umfassen. Gemäß einer Ausführungsform ist dann zwischen der ersten und der zweiten Spiegelfläche wenigstens eine optische Linse des Abbildungssystems angeordnet.

Um durch mehrfache Faltung des wenigstens einen Abbildungsstrahlenganges einen möglichst kompakten Aufbau des Mikroskopiesystems zu erzielen, kann das Abbildungssystem zur Ablenkung des wenigstens einen Abbildungsstrahlengangs weiter eine dritte Spiegelfläche und eine vierte Spiegelfläche aufweisen. Dann kann der wenigstens eine Abbildungsstrahlengang nacheinander an der ersten Spiegelfläche, der zweiten Spiegelfläche, der dritten Spiegelfläche und der vierten Spiegelfläche reflektiert sein.

Dabei kann es vorteilhaft sein, wenn die erste Spiegelfläche und die vierte Spiegelfläche relativ zueinander einen Winkel von zwischen 60° und 120° und bevorzugt zwischen 80° und 100° einschließen, sowie die zweite Spiegelfläche und die dritte Spiegelfläche relativ zueinander einen Winkel von zwischen 60° und 120° und bevorzugt zwischen 80° und 100° einschließen. Weiter können die dritte Spiegelfläche und die vierte Spiegelfläche relativ zueinander einen Winkel von im wesentlichen 60° einschließen. Der Grund ist, dass unter diesem Winkel angeordnete Spiegelflächen insgesamt wie ein Porro-System zweiter Art wirken. Folglich heben sich durch die Ablenkungen des wenigstens einen Abbildungsstrahlenganges an den Spiegelflächen hervorgerufene Seitenvertauschungen der Abbildung gegenseitig auf. Weiter ist eine durch die Ablenkungen der Abbildungsstrahlengänge insgesamt hervorgerufene Bildrotation gering bzw. nicht vorhanden.

Gemäß einer Ausführungsform wird bei einer derartigen Anordnung der ersten bis vierten Spiegelfläche der wenigstens eine Abbildungsstrahlengang so geführt, dass optische Achsen des wenigstens einen Abbildungsstrahlengangs in wenigstens einer ersten und zweiten Ebene verlaufen, die zueinander im wesentlichen parallel sind, sowie in wenigstens einer dritten Ebene verlaufen, welche zu den beiden ersten und zweiten Ebenen im wesentlichen senkrecht ist. Dabei soll die Bedingung von "im wesentlichen parallel" und "im wesentlichen senkrecht" als erfüllt angesehen werden, wenn die dritte Ebene die beiden ersten und zweiten Ebenen jeweils unter einem Winkel von zwischen 60° und 120° und bevorzugt zwischen 80° und 100° und besonders bevorzugt zwischen 85° und 95° und insbesondere 90° schneidet. Im vorliegenden Fall wird die erste Ebene durch optische Achsen von auf die erste Spiegelfläche ein- und ausfallenden Abbildungsstrahlenbündel des wenigstens einen Abbildungsstrahlenganges, die zweite Ebene durch optische Achsen der auf die vierte Spiegelfläche ein- und ausfallenden Abbildungsstrahlenbündel des wenigstens einen Abbildungsstrahlenganges, und die dritte Ebene durch optischen Achsen der auf die zweite (oder dritte) Spiegelfläche ein- und ausfallenden Abbildungsstrahlenbündel des wenigstens einen Abbildungsstrahlenganges aufgespannt.

Dabei kann der wenigstens eine Abbildungsstrahlengang zwischen der zweiten Spiegelfläche und der dritten Spiegelfläche frei von optischen Linsen sein.

Gemäß einer Ausführungsform kann der wenigstens eine Abbildungsstrahlengang auch an mehr oder weniger als vier Spiegelflächen abgelenkt sein, wobei die Spiegelflächen wechselseitig beliebige Winkel einschließen können. Eine dann auftretende Seitenvertauschung, Verzerrung oder Rotation der Abbildung kann dann durch eine entsprechende Korrektureinrichtung wahlweise optisch oder digital korrigiert werden.

Gemäß einer Ausführungsform weist das Mikroskopiesystem weiter einen zweiten Antrieb auf, welcher die zweite Spiegelfläche wahlweise um die zweite Schwenkachse verschwenkt. Zusätzlich oder alternativ kann das Mikroskopiesystem weiter einen ersten Antrieb aufweisen, welcher die erste Spiegelfläche wahlweise um die erste Schwenkachse verschwenkt. Dann ist die Steuerung bevorzugt ausgebildet, den ersten und/oder zweiten Antrieb zu steuern.

Es kann Vorteile bringen, wenn die erste Spiegelfläche zwischen der Objektebene und einer entlang des wenigstens einen Abbildungsstrahlengangs ersten optisch wirksamen Oberfläche des Abbildungssystems angeordnet ist. Der Grund ist, dass der abgelenkte Abbildungsstrahlengang dann z.B. keine optischen Linsen mehr durchlaufen muss, was bei einer großen Verkippung der ersten Spiegelfläche ggf. große optische Linsen erforderlich machen kann. Dabei wird unter einer optisch wirksamen Oberfläche eine Oberfläche mit einem Krümmungsradius von höchstens 10⁴ mm und bevorzugt höchstens 5*10³ mm und besonders bevorzugt höchstens 10³ mm verstanden. Somit sollen z.B. plane Filter oder Abdeckscheiben, die in den erfindungsgemäßen Mikroskopiesystem Verwendung finden können, hier nicht als optisch wirksame Oberfläche gelten.

Gemäß einer Ausführungsform ist der Abbildungsstrahlengang zwischen der ersten Spiegelfläche und der Objektebene frei von optisch wirksamen Elementen. Dabei werden unter optisch wirksamen Elementen derartige Elemente verstanden, durch deren Hinzufügen oder Entfernen ein Arbeitsabstand des Mikroskopiesystems um mehr als 0,5% und insbesondere mehr als 1% und weiter insbesondere um mehr als 2% und weiter insbesondere um mehr als 5% verändert wird. Filter oder Abdeckscheiben werden in diesem Zusammenhang nicht als optisch wirksame Elemente gesehen.

Allgemein kann das Mikroskopiesystem als Stereo-Mikroskopiesystem ausgebildet sein, bei dem das Abbildungssystem wenigstens ein Paar von Abbildungsstrahlengängen bereitstellt, die in der Objektebene einen Stereowinkel einschließen. Dann kann das Abbildungssystem ein erstes Teilsystem aufweisen, das eine Mehrzahl von Linsen umfasst, welche entlang einer gemeinsamen optischen Achse angeordnet und von beiden Abbildungsstrahlengängen des wenigstens einen Paars von Abbildungsstrahlengängen gemeinsam durchsetzt sind.

Weiter kann die erste und/oder zweite Spiegelfläche entlang der optischen Achse des ersten Teilsystems zwischen optischen Linsen des ersten Teilsystems angeordnet sein. Diese Anordnung der Spiegelflächen im ersten Teilsystem erleichtert es aufgrund der Verwendung von vergleichsweise großen gemeinsamen optischen Linsen für alle Abbildungsstrahlengänge, sicherzustellen, dass die Abbildungsstrahlengänge auch nach einem Verschwenken der Spiegelflächen noch durch die optischen Linsen hindurchgefädelt werden.

Dabei können wenigstens zwei Linsen des ersten Teilsystems entlang der optischen Achse relativ zueinander verlagerbar sein, um beispielsweise einen Arbeitsabstand und/oder eine Vergrößerung für alle Abbildungsstrahlengänge des Mikroskopiesystems gemeinsam einzustellen.

Allgemein kann das Abbildungssystem alternativ oder zusätzlich ein zweites Teilsystem aufweisen, dessen optischen Elemente eine Mehrzahl von Linsen umfassen, welche jeweils von lediglich einem Abbildungsstrahlengang des wenigstens einen Paars von Abbildungsstrahlengängen durchsetzt sind. Dabei können wenigstens zwei Linsen des zweiten Teilsystems entlang eines gemeinsamen Abbildungsstrahlengangs relativ zueinander verlagerbar sein.

Gemäß einer Ausführungsform kann das Mikroskopiesystem weiter ein Beleuchtungssystem mit einem Beleuchtungsstrahlengang zur Beleuchtung der Objektebene umfassen, wobei die erste und/oder zweite Spiegelfläche entlang des Beleuchtungsstrahlengangs angeordnet ist und wobei der Beleuchtungsstrahlengang wenigstens durch die erste und/oder zweite Spiegelfläche abgelenkt ist.

Indem auch der Beleuchtungsstrahlengang von der ersten und/oder zweiten Spiegelfläche abgelenkt ist, wird der Beleuchtungsstrahlengang bei einem Verschwenken der Spiegelfläche(n) zum Verlagern des Abbildungsfeldes des Abbildungssystems in der Objektebene automatisch nachgeführt. Hierfür ist der Beleuchtungsstrahlengang in geeigneter Weise (beispielsweise mittels eines halbdurchlässigen Spiegels) in den wenigstens einen Abbildungsstrahlengang eingekoppelt.

Alternativ kann das Mikroskopiesystem weiter ein Beleuchtungssystem mit einem Beleuchtungsstrahlengang zur Beleuchtung der Objektebene umfassen, wobei entlang des Beleuchtungsstrahlengangs wenigstens ein Beleuchtungsspiegel angeordnet ist, welcher in Abhängigkeit von dem durch die Steuerung ermittelten Versatz verschwenkbar ist. Hierdurch ist sichergestellt, dass der Beleuchtungsstrahlengang bei einem Versatz des Abbildungsfeldes des Abbildungssystems durch entsprechendes Verschwenken der wenigstens einen Spiegelfläche durch entsprechendes Verschwenken des Beleuchtungsspiegels automatisch nachgeführt wird.

Um neben der schnellen und genauen Positionierung des Abbildungsfeldes mittels der vorstehend beschriebenen Versatzeinrichtung auch eine grobe Positionierung des Abbildungsfeldes zu ermöglichen, kann das Mikroskopiesystem weiter ein Stativ umfassen, welches das Abbildungssystem trägt und wenigstens eine Verstellvorrichtung zur translatorischen Verlagerung des Abbildungssystems insgesamt aufweist. Dabei kann das Stativ beispielsweise drei oder mehr translatorische und zwei oder mehr rotatorische Freiheitsgrade aufweisen, um eine möglichst flexible Positionierung zu erlauben.

Ein Mikroskopiesystem mit den vorstehen beschriebenen Eigenschaften kann bevorzugt als Operationsmikroskop verwendet werden.

Im Folgenden werden bevorzugte Ausführungsformen der vorliegenden Erfindung unter Bezugnahme auf die beigefügten Zeichnungen beschrieben. In den Zeichnungen werden soweit möglich gleiche oder ähnliche Bezugszeichen verwendet, um auf gleiche oder ähnliche Elemente zu verweisen. Dabei zeigt
- Figur 1A: schematisch einen Strahlengang durch eine in eine Ebene entfaltete Anordnung wesentlicher Elemente eines Abbildungssystems eines Mikroskopiesystems gemäß einer bevorzugten ersten Ausführungsform der vorliegenden Erfindung;
- Figur 1B: schematisch eine Aufsicht von oben auf wesentliche Elemente des Abbildungssystems aus Figur 1A;
- Figur 1C: schematisch eine Seitenansicht der wesentlichen Elemente des Abbildungssystems aus Figur 1A;
- Figur 1D: schematisch eine perspektivische Ansicht einer räumlichen Anordnung der wesentlichen Elemente des Abbildungssystems aus Figur 1A;
- Figur 2: schematisch einen Strahlengang durch eine in eine Ebene entfaltete Anordnung wesentlicher Elemente eines Abbildungssystems eines Mikroskopiesystems gemäß einer zweiten Ausführungsform der vorliegenden Erfindung;
- Figur 3: schematisch einen Strahlengang durch eine Anordnung wesentlicher Elemente eines Abbildungssystems eines Mikroskopiesystems gemäß einer dritten Ausführungsform der vorliegenden Erfindung;
- Figur 4A: schematisch eine Seitenansicht eines Mikroskopiesystems gemäß der zweiten Ausführungsform der vorliegenden Erfindung; und
- Figur 4B: schematisch eine Seitenansicht eines Mikroskopiesystems gemäß einer vierten Ausführungsform der vorliegenden Erfindung.

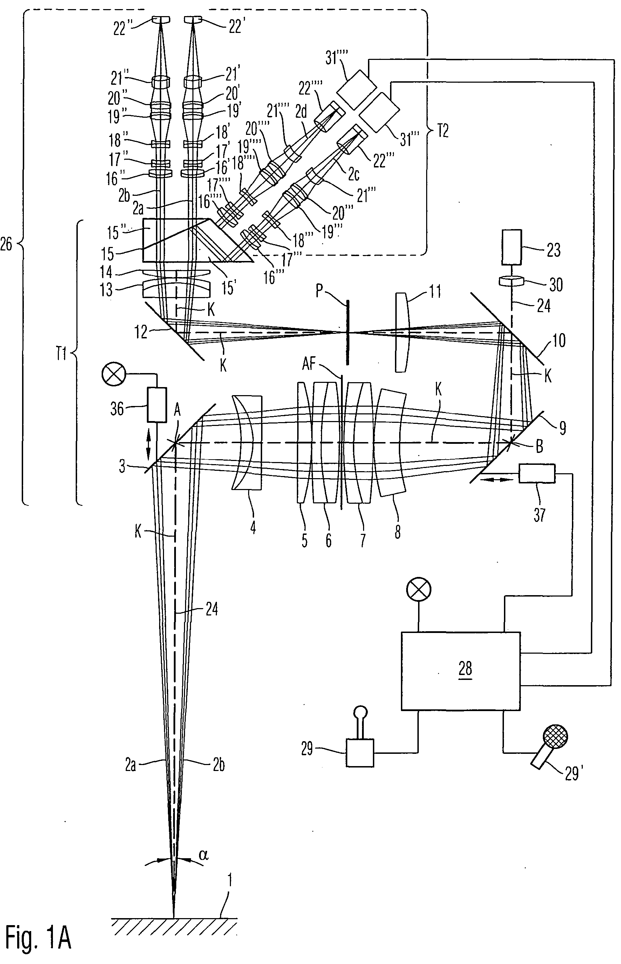

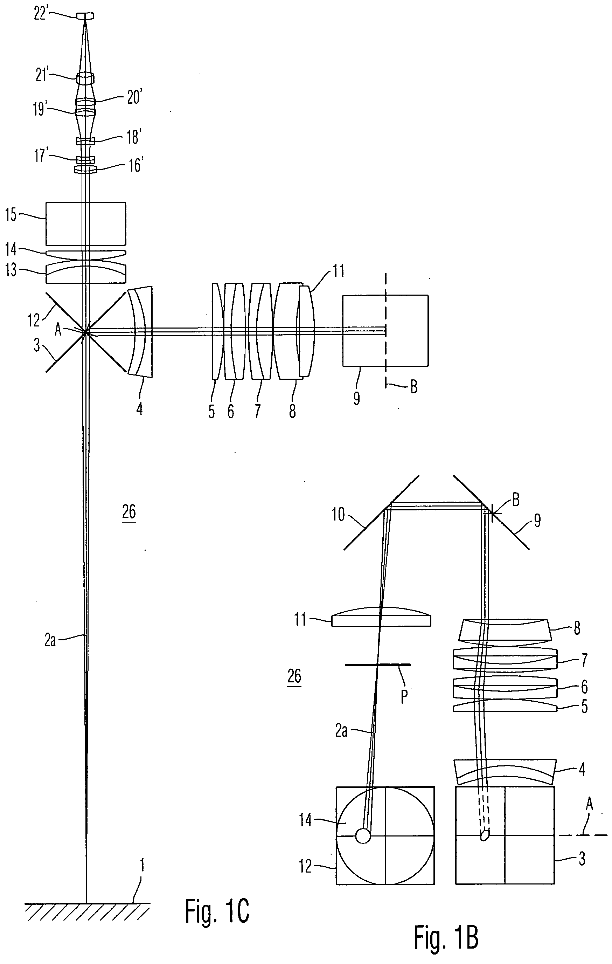

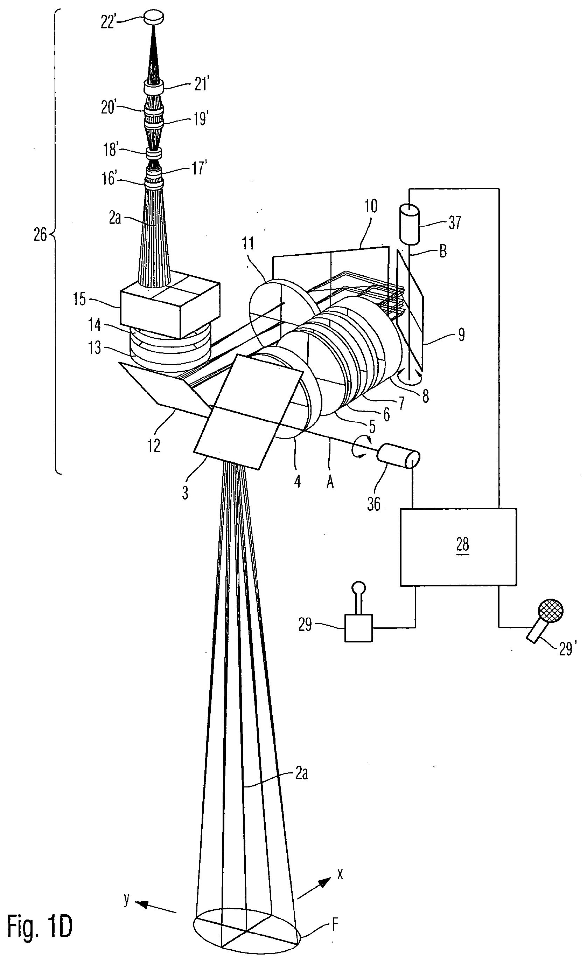

Im Folgenden wird eine erste bevorzugte Ausführungsform der vorliegenden Erfindung unter Bezugnahme auf die Figuren 1A, 1B, 1C und 1D näher erläutert.

Figur 1A zeigt schematisch einen Strahlengang durch eine in eine Ebene entfaltete Anordnung wesentlicher Elemente eines Abbildungssystems 26 eines Mikroskopiesystems gemäß der ersten Ausführungsform der vorliegenden Erfindung. Dabei zeigen die Figuren 1B, 1C und 1D schematisch verschiedene Ansichten auf wesentliche Elemente des Abbildungssystems des Mikroskopiesystems gemäß der bevorzugten Ausführungsform. In Figur 1A und 1D sind zusätzliche Elemente des Mikroskopiesystems schematisch in Form von Blockdiagrammen dargestellt.

Das Mikroskopiesystem gemäß der ersten bevorzugten Ausführungsform umfaßt ein optisches Abbildungssystem 26, welches zwei Paare von Abbildungsstrahlengängen 2a, 2b sowie 2c, 2d bereitstellt.

Die Abbildungsstrahlengänge 2a und 2b sowie die Abbildungsstrahlengänge 2c und 2d treffen sich jeweils paarweise in der Objektebene 1. Hauptstrahlen der Abbildungsstrahlengänge 2a, 2b und 2c, 2d schließen dabei jeweils paarweise einen Stereowinkel α ein. Somit bildet das Mikroskopiesystem ein Stereomikroskop. Dabei kann der in der Objektebene 1 von dem ersten Paar von Abbildungsstrahlengängen 2a, 2b eingeschlossene Stereowinkel α von dem (in den Figuren nicht gezeigten) Stereowinkel, der in der Objektebene 1 von dem zweiten Paar von Abbildungsstrahlengängen 2c, 2d eingeschlossen wird, verschieden sein. Die in der Objektebene 1 von den Abbildungsstrahlengängen 2a, 2b sowie 2c, 2d paarweise eingeschlossenen Stereowinkel können jedoch auch gleich groß sein. In Figur 1A beträgt der Stereowinkel α zwischen 4° und 6°. Die vorliegende Erfindung ist jedoch nicht auf den vorstehend angegebenen Winkelbereich beschränkt. Vielmehr ist es ausreichend, wenn der Stereowinkel ungleich Null Grad ist.

Wie in Figur 1D gezeigt, sind die Abbildungsstrahlengänge 2a, 2b, 2c und 2d ausgebildet, in der Objektebene 1 paarweise ein Abbildungsfeld F abzubilden. Das Abbildungsfeld F gibt den endlichen (d.h. nicht punktförmigen) Bereich eines in der Objektebene 1 angeordneten zu untersuchenden Objektes (nicht gezeigt) an, der von den Paaren von Abbildungsstrahlengängen 2a, 2b und 2c, 2d zu einem Zeitpunkt (d.h. gleichzeitig) abgebildet wird. Die Größe der Abbildungsfeldes F hängt von der Größe der optischen Elemente des Abbildungssystems 26 und der durch das Abbildungssystem 26 bereitgestellten Vergrößerung ab. In dem gezeigten Beispiel weist das Abbildungsfeld F einen Durchmesser von zwischen 10 mm und 100 mm auf. Die vorliegende Erfindung ist jedoch nicht auf eine bestimmte Größe des Abbildungsfeldes F beschränkt. Wesentlich ist lediglich, dass die Paare von Abbildungsstrahlengängen 2a, 2b und 2c, 2d zur Abbildung einer Fläche und nicht lediglich eines Punktes in der Objektebene 1 ausgebildet sind.

Das Abbildungssystem 26 wird von einem ersten optischen Teilsystem T1 und einem zweiten optischen Teilsystem T2 gebildet, welche Teilsysteme T1 und T2 jeweils eine Mehrzahl optischer Elemente aufweisen.

Das erste Teilsystem T1 weist entlang einer gemeinsamen optischen Achse K ein erstes optisches Umlenkelement mit einer ersten optischen Spiegelfläche 3, eine erste, zweite, dritte, vierte und fünfte optische Linse 4, 5, 6, 7 und 8, ein zweites optisches Umlenkelement mit einer zweiten optischen Spiegelfläche 9, ein drittes optisches Umlenkelement mit einer dritten optischen Spiegelfläche 10, eine sechste optische Linse 11, ein viertes optisches Umlenkelement mit einer vierten optischen Spiegelfläche 12, eine siebte und achte optische Linse 13 und 14 sowie Prismenteile 15', 15" einer Strahlteileranordnung 15 auf.

Dabei werden die Linsen 4, 5, 6, 7, 8, 11, 13 und 14 des ersten Teilsystems T1 von den vier Abbildungsstrahlengängen 2a, 2b, 2c und 2d gemeinsam durchsetzt. Weiter ist zwischen der dritten und vierten optischen Linse 6, 7 eine afokale Schnittstelle AF angeordnet, in dem die Abbildungsstrahlengänge 2a, 2b, 2c und 2d jeweils nach Unendlich abgebildet werden. Das Vorsehen der afokalen Schnittstelle AF ermöglicht einen modularen Aufbau des Abbildungssystems 26. Es wird jedoch betont, dass die afokale Schnittstelle AF zur Verwirklichung der Erfindung nicht erforderlich ist.

Die Abbildungsstrahlengänge 2a, 2b, 2c und 2d werden nacheinander an der ersten Spiegelfläche 3, der zweiten Spiegelfläche 9, der dritten Spiegelfläche 10 und der vierten Spiegelfläche 12 reflektiert und so abgelenkt. Wie besonders gut aus Figur 1D ersichtlich, schließen dabei Normalenvektoren der durch die erste Spiegelfläche 3 und die vierte Spiegelfläche 12 aufgespannten Ebenen relativ zueinander einen variablen Winkel von zwischen 70° und 110° ein. Weiter schließen Normalenvektoren der durch die zweite Spiegelfläche 9 und die dritte Spiegelfläche 10 jeweils aufgespannten Ebenen relativ zueinander einen variablen Winkel von zwischen 70° und 110° ein. Dabei ist die vorliegende Erfindung jedoch nicht auf einen derartigen Winkelbereich beschränkt. Normelenvektoren der durch die dritte Spiegelfläche 10 und die vierte Spiegelfläche 12 aufgespannten Ebenen schließen relativ zueinander einen konstanten Winkel von im wesentlichen 60° ein. Dabei wird in dieser Anmeldung unter "im wesentlichen 60°" eine Abweichung von 60° um höchstens 5° und bevorzugt höchstens 2° und besonders bevorzugt höchstens 1° verstanden.

Diese Anordnung der ersten bis vierten Spiegelfläche 3, 9, 10 und 12 wirkt optisch insgesamt wie ein Porro-System zweiter Art. Das heißt, dass die erste bis vierte Spiegelfläche 3, 9, 10 und 12 sowohl eine Bildumkehr als auch eine Pupillenvertauschung bewirken. Weiter wird durch diese Anordnung der Spiegelflächen 3, 9, 10 und 12 ein besonders kompakter Aufbau des Abbildungssystems 26 erzielt.

Die erste bis fünfte Linse 4, 5, 6, 7 und 8 sind zwischen dem ersten Umlenkelement mit der ersten Spiegelfläche 3 und dem zweiten Umlenkelement mit der zweiten Spiegelfläche 9 angeordnet. Die sechste Linse 11 ist zwischen dem dritten Umlenkelement mit der dritten Spiegelfläche 10 und dem vierten Umlenkelement mit der vierten Spiegelfläche 12 angeordnet. Die siebte und achte Linse 13 und 14 sind zwischen dem vierten Umlenkelement mit der vierten Spiegelfläche 12 und der Strahlteileranordnung 15 angeordnet.

Somit ist der Strahlengang zwischen dem zweiten Umlenkelement mit der zweiten Spiegelfläche 9 und dem dritten Umlenkelement mit der dritten Spiegelfläche 10 frei von optischen Linsen. Weiter ist die erste Spiegelfläche 3 zwischen der Objektebene 1 und der ersten Linse 4 und damit zwischen der Objektebene 1 und der ersten optisch wirksamen Oberfläche des Abbildungssystems 26 entlang der Abbildungsstrahlengänge 2a, 2b, 2c und 2d angeordnet. Dabei wird unter einer optisch wirksamen Oberfläche eine Oberfläche mit einem Krümmungsradius von höchstens 10⁴ mm und bevorzugt höchstens 5*10³ mm und besonders bevorzugt höchstens 10³ mm verstanden. Somit sollen z.B. plane Filter oder Abdeckscheiben hier nicht als optisch wirksame Oberfläche gelten. Dabei ist die vorliegende Erfindung jedoch nicht auf eine derartige Anordnung der ersten Spiegelfläche beschränkt. Somit kann alternativ die erste wirksame Oberfläche des Abbildungssystems auch zwischen der ersten Spiegelfläche und der Objektebene angeordnet sein (nicht gezeigt).

Das die erste Spiegelfläche 3 aufweisende erste Umlenkelement ist um eine erste Schwenkachse A verschwenkbar. Hierfür ist das erste Umlenkelement mit einem ersten Antrieb 36 verbunden. In der gezeigten bevorzugten Ausführungsform ist der erste Antrieb 36 ein Schrittmotor, dessen Motorachse direkt die erste Schwenkachse A bildet, welche das die erste Spiegelfläche 9 aufweisende erste Umlenkelement trägt. Wie am besten aus Figur 1D ersichtlich, führt ein Verschwenken der ersten Spiegelfläche 3 mittels des ersten Antriebs 37 um die erste Schwenkachse A zu einer translatorischen Verlagerung des Abbildungsfeldes F des Abbildungssystems 26 in der Objektebene 1 in X-Richtung. Weiter liegt in Figur 1D die erste Schwenkachse A in einem Bereich, in dem die optische Achse K auf die erste Spiegelfläche 3 trifft. Dies ist in Figur 1D in der Mitte der ersten Spiegelfläche 3.

Auf ähnliche Weise ist das die zweite Spiegelfläche 9 aufweisende zweite Umlenkelement um eine zweite Schwenkachse B verschwenkbar, welche zweite Schwenkachse B von der ersten Schwenkachse A verschieden ist. Hierfür ist das zweite Umlenkelement mit einem zweiten Antrieb 37 verbunden, der in der gezeigten Ausführungsform durch einen Schrittmotor gebildet wird, dessen Motorachse das zweite Umlenkelement trägt und die zweite Schwenkachse B festlegt. Wie aus Figur 1D ersichtlich, führt ein Verschwenken der zweiten Spiegelfläche 9 mittels des zweiten Antriebs 38 zu einer translatorischen Verlagerung des Abbildungsfeldes F des Abbildungssystem 26 in der Objektebene 1 in Y-Richtung. Weiter liegt in Figur 1D die zweite Schwenkachse B in einem Bereich, in dem die optische Achse K auf die zweite Spiegelfläche 9 trifft. Dies ist in Figur 1D in der Mitte der zweiten Spiegelfläche 9.

In der ersten bevorzugten Ausführungsform schließt die erste Schwenkachse A mit einer ersten Ablenkungsebene, die von der auf die erste Spiegelfläche 3 einfallenden und von der ersten Spiegelfläche 3 ausfallenden optischen Achse K der Abbildungsstrahlengänge 2a, 2b, 2c, 2d aufgespannt ist, einen Winkel von im wesentlichen 90° ein. Dabei wird die optische Achse K durch die Linsen 4 bis 8, 11, 13, 14 des ersten Teilsystems T1 festgelegt. Es ist offensichtlich, dass die optische Achse K in der ersten Ausführungsform nicht entlang einer einzigen Geraden verläuft, sondern durch die Spiegelflächen 3, 9, 10, 12 abgeknickt ist. Weiter schließt auch die zweite Schwenkachse B mit einer zweiten Ablenkungsebene, die von der auf die zweite Spiegelfläche 9 einfallenden und von der zweiten Spiegelfläche 9 ausfallenden optischen Achse K der Abbildungsstrahlengänge 2a, 2b, 2c, 2d aufgespannt ist, einen Winkel von im wesentlichen 90° ein. Anstelle der optischen Achse K können hier auch Hauptstrahlen der Abbildungsstrahlengänge 2a bis 2d als Bezug verwendet werden. Ersichtlich müssen die Hauptstrahlen der Abbildungsstrahlengänge 2a, 2b, 2c und 2d hierzu in einer gemeinsamen Ebene liegen. Zudem ist die zweite Schwenkachse B zu der ersten Ablenkungsebene im wesentlichen parallel angeordnet. Dies bedeutet, dass die zweite Schwenkachse B zu der ersten Ablenkungsebene parallel ist, oder dass die zweite Schwenkachse B die erste Ablenkungsebene schneidet und dabei mit der ersten Ablenkungsebene einen Winkel von kleiner 5° und bevorzugt kleiner 2° einschließt, oder dass die zweite Schwenkachse B und die erste Ablenkungsebene zusammenfallen. In der gezeigten ersten Ausführungsform liegt die zweite Schwenkachse B in der ersten Ablenkungsebene.

Gemeinsam bilden das erste und zweite Umlenkelement und der erste und zweite Antrieb 36, 37 so eine Versatzeinrichtung, um das Abbildungsfeld F des Abbildungssystems 26 durch kombiniertes Antreiben des ersten und zweiten Antriebs 36, 37 und entsprechendes Verschwenken der ersten und zweiten Spiegelfläche 3, 9 in der Objektebene 1 translatorisch in eine beliebige Richtung zu verlagern. Dabei sind der Verlagerung durch die Optik des Abbildungssystems 26 Grenzen gesetzt, da die Abbildungsstrahlengänge 2a, 2b, 2c, 2d nicht nach außerhalb der von ihnen durchsetzten optischen Linsen 4, 5, 6, 7, 8 des Abbildungssystems 26 verlagert werden dürfen.
Der erste und zweite Antrieb 36, 37 ist jeweils mit einer Steuerung 28 verbunden. Der besseren Übersicht wegen ist die Verbindungsleitung zwischen dem ersten Antrieb 36 und der Steuerung 28 in Figur 1A nicht vollständig gezeigt. Die Steuerung 28 ist mit Benutzerschnittstellen in Form eines Joysticks 29 und eines Mikrophons 29' verbunden.
Alternativ können jedoch auch beliebige andere Benutzerschnittstellen verwendet werden, die Steuerbefehle von einem Benutzer beispielsweise in Form von Sprache und/oder einer Augenbewegung und/oder einer Fußbewegung und/oder einer Kopfbewegung und/oder einer Handbewegung des Benutzers empfangen und (bevorzugt in digitaler Form oder in analoger Form) an die Steuerung ausgeben. Dabei können die Steuerbefehle beispielsweise einen absoluten Versatz, und/oder einen relativen Versatz und/oder lediglich eine Richtung eines Versatzes in Verbindung mit einer Zeitspanne angeben.

In Abhängigkeit von einem über den Joystick 29 oder das Mikrophon 29' empfangenen Befehl eines Benutzers ermittelt die Steuereinrichtung 28 einen gewünschten Versatz des Abbildungsfeldes F in der Objektebene 1 und steuert die ersten und zweiten Antriebe 36, 37 entsprechend.

Zur Ermittlung einer für einen gewünschten Versatz des Abbildungsfeldes F erforderlichen Winkelstellung der ersten und zweiten Spiegelfläche 3, 9 kann die Steuereinrichtung 28 auf eine Tabelle zurückgreifen, in welcher in Abhängigkeit von einer jeweiligen Gesamtvergrößerung des Mikroskopiesystems die für einen gewünschten Versatz erforderlichen Winkelstellungen der ersten und zweiten Spiegelfläche 3, 9 gespeichert sind. Alternativ oder zusätzlich kann sich die Steuerung auch einer entsprechenden Umrechnungs-Formel bedienen oder die ersten und zweiten Antriebe 36, 37 direkt in Abhängigkeit von einem empfangenen Befehl eines Benutzers steuern.

Wie bereits betont, sind die optischen Linsen 4-8 des ersten Teilsystems T1 entlang der gemeinsamen optischen Achse K angeordnet. Dabei ist die erste Linse 4 relativ zu der zweiten Linse 5 sowie die dritte Linse 6 relativ zu der vierten Linse 7 entlang der optischen Achse K verlagerbar, um einen Abstand der Objektebene 1 von dem Abbildungssystem 26 des Mikroskopiesystems und damit einen Arbeitsabstand und/oder eine Vergrößerung der Abbildung eines in der Objektebene 1 anordenbaren Objektes zu ändern. Gleichzeitig ist durch geeignete Wahl der Systemdaten dieser optischen Linsen 4, 5, 6 und 7 sichergestellt, daß die Abbildungsstrahlengänge 2a und 2b sowie 2c und 2d auch nach einer Verlagerung der Linsen 4, 5, 6, 7 in der Objektebene paarweise den von Null verschiedenen Stereowinkel α einschließen.

Auch das zweite Teilsystem T2 des Abbildungssystems 26 weist eine Vielzahl von optischen Elementen 16' - 22', 16" - 22", 16"' - 22"' und 16"" - 22"" auf, in denen die Abbildungsstrahlengänge 2a, 2b, 2c und 2d jedoch anders als im ersten Teilsystem T1 jeweils getrennt geführt werden. Dies bedeutet, daß die optischen Linsen 16' - 21', 16" - 21", 16'" - 21"' und 16"" - 21"" jeweils von je einem Abbildungsstrahlengang 2a, 2b, 2c oder 2d durchsetzt sind.

Jeder Abbildungsstrahlengang 2a und 2b des zweiten Teilsystems T2 weist eine nur schematisch gezeigten stereoskopischen Einblick aus der Tubusoptik mit den Okularen 22', 22" für eine direkte visuelle Beobachtung durch einen Benutzer auf.

Jeder Abbildungsstrahlengang 2c und 2d des zweiten Teilsystems T2 weist einen Kameraadapter 22"' und 22"" für eine Digitalkamera 31'" und 31"" zur Erzeugung von Bilddaten auf. Anstelle getrennter Kameras 31"' und 31"" kann auch eine Stereokamera verwendet werden. Die Kameras 31"' und 31"" sind jeweils mit der Steuerung 28 verbunden.

Die Steuerung 28 empfängt die von den Kameras 31"', 31"" jeweils erzeugten Bilddaten und detektiert in den Bilddaten automatisch die Position eines Markers (nicht gezeigt). Bei diesem Marker kann es sich beispielsweise je nach Anwendungsfall / betrachtetem Objekt um ein separates, in der Objektebene eigens angeordnetes Element wie beispielsweise einen charakteristisch ausgebildeten Aufkleber handeln. Alternativ kann es sich bei dem Marker beispielsweise auch um ein in den Bilddaten automatisch identifiziertes charakteristisches Element des Objektes selber wie z.B. ein in den Bilddaten abgebildetes chirurgisches Element (oder Instrument) oder ein bestimmtes Körperteil eines Patienten handeln.

In der gezeigten Ausführungsform ist die Steuerung über den Joystick 29 und/oder das Mikrophon 29' in einen Betriebszustand schaltbar, in dem die Steuerung einen gewünschten Versatz nicht direkt anhand eines über den Joystick 29 und/oder das Mikrophon 29' empfangenen Befehls, sondern indirekt anhand der in den Bilddaten detektierten Position des Markers ermittelt. In der Folge steuert die Steuerung 28 die ersten und zweiten Antriebe 36, 37 in Abhängigkeit von der detektierten Position des Markers. In dieser bevorzugten Ausführungsform steuert die Steuerung 28 die ersten und zweiten Antriebe 36, 37 so, dass die detektierte Position des Markers in den Bilddaten im wesentlichen konstant bleibt. Dies bedeutet, dass die Steuerung die erste und zweite Spiegelfläche 3, 9 mittels der ersten und zweiten Antriebe 36, 37 so verschwenkt, dass ein beispielsweise in der Mitte der Bilddaten detektierter Marker auch nach einer Verlagerung des Markers relativ zu der Objektebene in der Mitte der Bilddaten verbleibt. Dabei soll unter "im wesentlichen konstant" verstanden werden, dass sich die relative Position des Markers in den Bilddaten um nicht mehr als 30% eines Durchmessers des Abbildungsfeldes F und bevorzugt um nicht mehr als 10% des Durchmessers des Abbildungsfeldes F verändert.

Weiter sind jeweils drei Abstände zwischen vier Linsen 16' - 19', 16" - 19", 16'" - 19'" und 16"" - 19"", die in einem jeweiligen Abbildungsstrahlengang 2a, 2b, 2c und 2d entlang einer gemeinsamen optischen Achse (nicht gezeigt) angeordnet sind, relativ zueinander verlagerbar, um eine Änderung einer Vergrößerung der von dem zweiten Teilsystem T2 in den jeweiligen Abbildungsstrahlengängen 2a, 2b, 2c und 2d jeweils bewirkten Abbildung zu bewirken.

Zur paarweisen Trennung der Abbildungsstrahlengänge 2a, 2b, 2c und 2d ist ein physikalischer Strahlteiler 15 vorgesehen, der eine teilweise transparente Spiegelfläche aufweist, welche von einem ersten Paar von Abbildungsstrahlengängen 2a und 2b durchsetzt ist und an welcher ein zweites Paar von Abbildungsstrahlengängen 2c und 2d reflektiert ist.

Weiter stellt das Mikroskopiesystem gemäß der ersten bevorzugten Ausführungsform einen Sekundärstrahlengang 24 bereit, welcher die dritte Spiegelfläche 10 des dritten Umlenkelements in einem zentralen Bereich durchsetzt. Dieser zentrale Bereich kann vorzugsweise zwischen Strahlquerschnittsflächen der Abbildungsstrahlengänge 2a, 2b, 2c und 2d liegen. Hierfür weist die dritte Spiegelfläche 10 zumindest bereichsweise eine Transparenz für Strahlung des Sekundärstrahlengangs 24 auf, die größer ist als eine Transparenz für Strahlung der Abbildungsstrahlengänge 2a, 2b, 2c, 2d. Die Einkopplung des Sekundärstrahlenganges 24 kann alternativ jedoch auch auf eine andere Weise erfolgen.

In Figur 1A wird der Sekundärstrahlengang 24 durch eine Beleuchtungsoptik 30 eines Beleuchtungssystems gebildet, wobei das Beleuchtungssystem weiter eine Strahlungsquelle 23 umfaßt. Dieses Beleuchtungssystem ist nicht Teil des Abbildungssystems 26.

Alternativ kann zusätzlich oder anstelle des die Beleuchtungsoptik 30 und die Strahlungsquelle 23 umfassenden Beleuchtungssystems auch ein Infrarot-Beobachtungssystem (nicht gezeigt) mit einer Infrarot-Abbildungsoptik und einer Infrarot-Kamera vorgesehen sein, wobei die Infrarot-Abbildungsoptik den Sekundärstrahlengang 24 bereitstellt.

Weiter kann zusätzlich oder anstelle des Beleuchtungssystems auch ein Laser (nicht gezeigt) mit einem Strahlführungssystem (nicht gezeigt), welches den Sekundärstrahlengang 24 bereitstellt, vorgesehen sein. Ein derartiger Laser ermöglicht eine Therapie beispielsweise zur Krebsbehandlung.

Da der Sekundärstrahlengang 24 nacheinander von der zweiten Spiegelfläche 9 und der ersten Spiegelfläche 3 reflektiert und so abgelenkt wird, wird der Sekundärstrahlengang bei einer Verschwenkung der zweiten Spiegelfläche 9 und der ersten Spiegelfläche 3 zwecks translatorischer Verschiebung des Abbildungsfeldes F in der Objektebene 1 automatisch nachgeführt. Das in Figur 1A gezeigte Mikroskopiesystem weist somit für jede Stellung der Spiegelflächen eine 0°-Beleuchtung für ein in der Objektebene 1 anordenbares Objekt auf. Über weite Bereiche überlappen sich in Figur 1A die optische Achse K des ersten Teilsystems T1 und der Sekundärstrahlengang 24.

In der vorstehend beschriebenen ersten bevorzugten Ausführungsform ist das erste, zweite, dritte und vierte Umlenkelement jeweils ein optischer Spiegel. Alternativ können die Umlenkelemente jedoch beispielsweise auch Prismen mit jeweils wenigstens einer Spiegelfläche sein. Weiter können das erste, zweite, dritte und vierte Umlenkelement wahlweise jeweils mehrere Spiegelflächen zur Ablenkung der Abbildungsstrahlengänge 2a, 2b, 2c und 2d aufweisen. Zudem können mehr oder weniger als zwei Paar von Abbildungsstrahlengängen vorgesehen sein.

Weiter sind in der vorstehend beschriebenen ersten bevorzugten Ausführungsform die erste, zweite, dritte und vierte Spiegelfläche so angeordnet, dass die gemeinsame optische Achse K des ersten Teilsystems T1 durch die Spiegelflächen so gefaltet wird, dass sie in wenigstens einer ersten und zweiten Ebene, die zueinander im wesentlichen parallel sind, sowie in wenigstens einer dritten Ebene, welche zu den beiden ersten und zweiten Ebenen im wesentlichen senkrecht ist, liegt. Dabei sollen die Bedingungen von "im wesentlichen parallel" und "im wesentlichen senkrecht" als erfüllt angesehen werden, wenn die dritte Ebene die beiden ersten und zweiten Ebenen jeweils unter einem Winkel von zwischen 60° und 120° und bevorzugt zwischen 80° und 100° und besonders bevorzugt zwischen 85° und 95° und insbesondere 90° schneidet. Im vorliegenden Fall wird die erste Ebene durch die auf die erste Spiegelfläche 3 ein- und ausfallende optische Achse K, die zweite Ebene durch die auf die vierte Spiegelfläche 12 ein- und ausfallende optische Achse K, und die dritte Ebene durch die auf die zweite (oder dritte) Spiegelfläche 9 (oder 10) ein- und ausfallende optische Achse K aufgespannt.

Das Mikroskopiesystem gemäß der ersten bevorzugten Ausführungsform eignet sich besonders gut zur Verwendung als Operationsmikroskop. Der Grund ist, dass es einem Benutzer durch Verschwenken der ersten und zweiten Spiegelfläche 3, 9 mittels der Steuerung 28 auf besonders einfache, schnelle, zuverlässige und vibrationsfreie Weise möglich ist, das Abbildungsfeld F in der Objektebene 1 zu verlagern. Weiter kann die Steuerung 28 eine automatische Bildstabilisierung und Bildnachführung bewirken, indem sie die ersten und zweiten Spiegelflächen 3, 9 automatisch in Abhängigkeit von der in Bilddaten detektierten Position des Markers steuert.

In den Figuren 1B bis 1D wurde der besseren Übersichtlichkeit halber nur jeweils ein Abbildungsstrahlengang 2a dargestellt. Aus dem gleichen Grund sind die Abbildungsstrahlengänge 2c und 2d in Figur 1A nicht vollständig gezeigt. Zudem wurde in den Figuren 1B, 1C und 1D auf eine Darstellung des Beleuchtungssystems und der optischen Achse K des ersten Teilsystems T1 verzichtet. Figur 1D zeigt schematisch eine perspektivische Ansicht, um (im Gegensatz zu der in Figur 1A in eine Ebene entfalteten Anordnung) die tatsächliche räumliche Anordnung wesentlicher Elemente des Abbildungssystems 26 des Mikroskopiesystems gemäß der ersten bevorzugten Ausführungsform zu verdeutlichen.

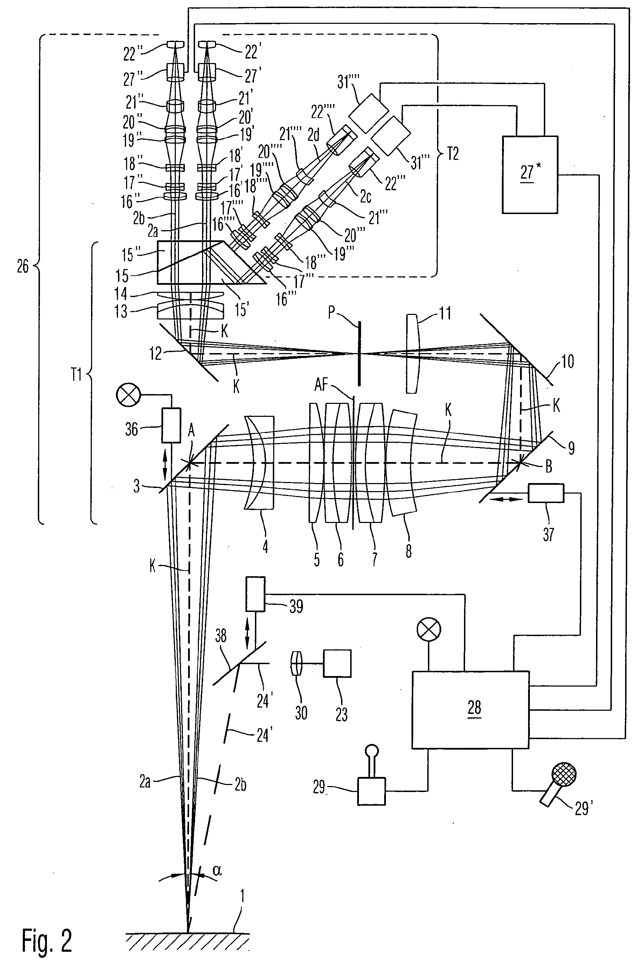

Im Folgenden wird unter Bezugnahme auf Figur 2 eine zweite Ausführungsform eines Mikroskopiesystems gemäß der vorliegenden Erfindung beschrieben. Dabei zeigt Figur 2 schematisch einen Strahlengang durch eine in eine Ebene entfaltete Anordnung wesentlicher Elemente eines Abbildungssystems des Mikroskopiesystems. Zusätzliche Elemente des Mikroskopiesystems sind schematisch in Form von Blockdiagrammen dargestellt. Da der Aufbau des Mikroskopiesystems gemäß der zweiten Ausführungsform dem Aufbau des Mikrokopiesystems gemäß der vorstehend ausführlich beschriebenen ersten Ausführungsform in vielen Teilen entspricht, werden nur die Unterschiede zwischen der ersten und zweiten Ausführungsform erläutert.

Durch das wahlweise Verschwenken der ersten und zweiten Spiegelfläche 3, 9 wird eine Rotation der durch das Abbildungssystem 26 erstellten Abbildung des in der Objektebene 1 des Mikroskopiesystems anordenbaren Objekts und damit des Abbildungsfeldes F hervorgerufen, wenn die erste und zweite Drehachse A, B der ersten und zweiten Spiegelfläche 3, 9 nicht jeweils mit einer jeweiligen Ablenkungsebene, die von der auf die jeweilige Spiegelfläche 3, 9 einfallenden und von der jeweiligen Spiegelfläche 3, 9 ausfallenden optischen Achse K und/oder Hauptstrahlen der Abbildungsstrahlengänge 2a bis 2d des ersten Teilsystems T1 aufgespannt ist, einen Winkel von im wesentlichen 90° einschließen.

Um eine beliebige Anordnung der Drehachsen der verschwenkbaren Spiegelflächen 3, 9 zu ermöglichen, weist das Mikroskopiesystem gemäß der zweiten Ausführungsform zusätzlich eine Kompensationseinrichtung auf, welche eine (zusätzliche) Rotation der durch das Abbildungssystem 26 erzeugten Abbildung des Abbildungsfeldes F bewirkt.

Hierfür sind in dem ersten und zweiten Abbildungsstrahlengang 2a, 2b jeweils Kompensationseinrichtungen in Form von einstellbaren Prisma-Anordnungen 27' bzw. 27" vorgesehen. Die einstellbaren Prisma-Anordnungen 27' und 27" sind mit der Steuerung 28 verbunden und weisen jeweils wenigstens eine und bevorzugt jeweils wenigstens zwei Spiegelflächen auf, die zur optischen Rotation der durch das Abbildungssystem 26 bereitgestellten Abbildung relativ zueinander verdrehbar sind. Die vorliegende Erfindung ist jedoch nicht auf einen derartigen Aufbau der optischen Kompensationseinrichtung beschränkt.

Die Steuerung 28 steuert die einstellbaren Prisma-Anordnungen 27' und 27" in Abhängigkeit von einer Steuerung der ersten und zweiten Antriebe 36, 37 so, dass eine durch ein Verschwenken der ersten und zweiten Spiegelfläche 3, 9 hervorgerufene Rotation der durch das Abbildungssystem 26 bereitgestellten Abbildung des Objektes durch eine von den einstellbaren Prisma-Anordnungen 27', 27" bewirkten entgegengesetzten Rotation der Abbildung aufgehoben wird.

Weiter weist das Mikroskopiesystem für die Kompensation einer Rotation der durch das Abbildungssystem bereitgestellten Abbildung des Objektes in den Abbildungsstrahlengängen 2c und 2d eine weitere Kompensationseinrichtung in Form eines externen Grafikprozessors 27* auf. Der Grafikprozessor 27* ist mit den Kameras 31"' und 31"" und mit der Steuerung 28 verbunden. Die Steuerung 28 steuert der Grafikprozessor 27* in Abhängigkeit von einer Steuerung der ersten und zweiten Antriebe 36, 37 so, dass eine durch ein Verschwenken der ersten und zweiten Spiegelfläche 3, 9 hervorgerufene Rotation der durch das Abbildungssystem 26 bereitgestellten Abbildung des Objektes durch eine von dem Grafikprozessor 27* mittels elektronischer Bildverarbeitung bewirkte Rotation der Abbildung unter zusätzlicher Anpassung der Stereobasis aufgehoben wird. Dabei berücksichtigt die Steuerung 28 sowohl das Maß der Verschwenkung der jeweiligen Spiegelfläche 3, 9 als auch die Orientierung der jeweiligen Schwenkachse A, B relativ zu dem jeweils abgelenkten Abbildungsstrahlengang 2a, 2b, 2c und 2d bzw. der optischen Achse K des ersten Teilsystems T1. Alternativ kann die Kompensation beispielsweise auch durch vorzugsweise gesteuerte mechanische Drehung des Kamerapaares 31"', 31"" erfolgen.

Weiter wird der Beleuchtungsstrahlengang 24' des Mikroskopiesystems gemäß der zweiten Ausführungsform nicht durch die verschwenkbaren ersten und zweiten Spiegelflächen 3, 9 abgelenkt. Um den Beleuchtungsstrahlengang 24' bei einer Verlagerung des Abbildungsfeldes F in der Objektebene 1 in Folge eines Verschwenkens der ersten und zweiten Spiegelfläche 3, 9 dennoch nachführen zu können, weist das Mikroskopiesystem einen zusätzlichen Beleuchtungsspiegel 38 auf. Der Beleuchtungsspiegel 38 lenkt den Beleuchtungsstrahlengang 24' ab und ist in der gezeigten Ausführungsform mittels eines dritten Antriebs 39 in Form eines Schrittmotors um zwei zueinander orthogonale Achsen, die beide in einer von dem Beleuchtungsspiegel 38 aufgespannten Ebene liegen, verschwenkbar. Die beiden Achsen des Beleuchtungsspiegels 38 schneiden sich bevorzugt in einem Bereich, in dem der Beleuchtungsstrahlengang 24' auf den Beleuchtungsspiegel 38 trifft. Dabei ist der dritte Antrieb 39 mit der Steuerung 28 verbunden. Die Steuerung 28 steuert den dritten Antrieb 39 in Abhängigkeit von einer Ansteuerung der ersten und zweiten Antriebe 36, 37 so, dass der Beleuchtungsspiegel 38 bei einem Verschwenken der ersten und zweiten Spiegelflächen 3, 9 zwecks Verlagerung des Abbildungsfeldes F in der Objektebene 1 so verschwenkt wird, dass der Beleuchtungsstrahlengang 24' der Verlagerung der Abbildungsfeldes F folgt.

Auch in dieser zweiten Ausführungsform kann der Beleuchtungsstrahlengang 24' alternativ ein beliebiger Sekundärenstrahlengang zur Beobachtung und/oder Beeinflussung eines in der Objektebene 1 anordenbaren Objektes sein.

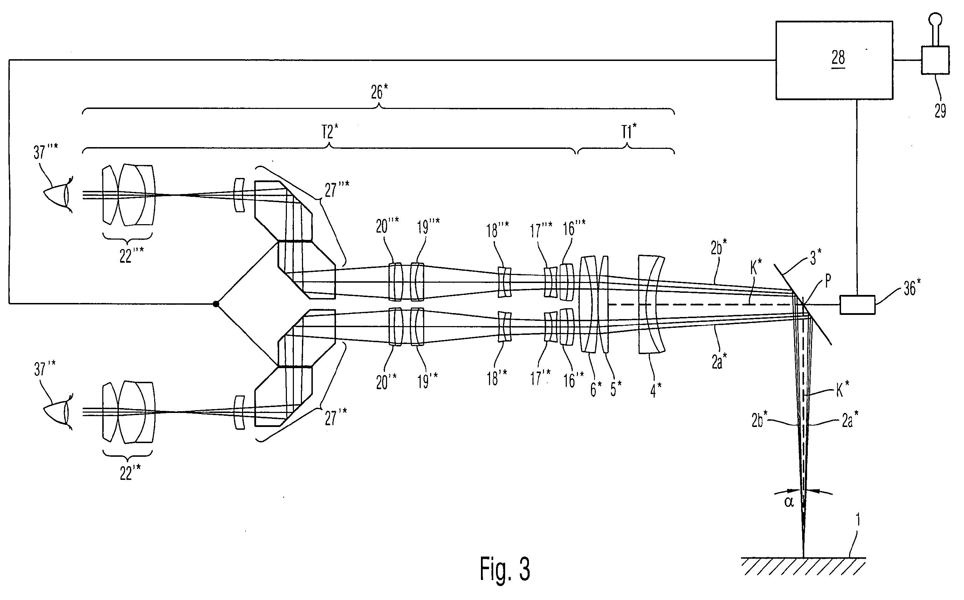

Im Folgenden wird unter Bezugnahme auf Figur 3 eine Ausführungsform eines Mikroskopiesystems beschrieben, die nicht Gegenstand der Ansprüche ist. Dabei zeigt Figur 3 schematisch einen Strahlengang durch wesentliche Elemente des Mikroskopiesystems, wobei zusätzliche Elemente des Mikroskopiesystems schematisch in Form von Blockdiagrammen dargestellt sind.

Auch das Mikroskopiesystem gemäß der dritten Ausführungsform weist ein Abbildungssystem 26* auf, um ein in einer Objektebene 1 angeordnetes Objekt (nicht gezeigt) abzubilden. Dabei setzt sich das Abbildungssystem 26* (wie in der vorstehend beschriebenen ersten und zweiten Ausführungsform) aus einem ersten Teilsystem T1* mit mehreren optischen Linsen 4*, 5* und 6*, in denen Abbildungsstrahlengänge 2a*, 2b* gemeinsam geführt werden, und einem zweiten Teilsystem T2* mit mehreren optischen Linsen 16'*-20'*, 22'*, 16''*-20"*, 22"*, in denen die Abbildungsstrahlengänge 2a*, 2b* getrennt geführt werden, zusammen. Auch hier sind Linsen des ersten und zweiten Teilsystems T1*, T2* zur Anpassung eines Arbeitsabstandes bzw. zur Änderung der Abbildungsvergrößerung relativ zueinander verlagerbar. Auf eine nähere Beschreibung dieser Elemente wird verzichtet.

Anders als in den vorangegangenen Ausführungsformen sind nur zwei Abbildungsstrahlengänge 2a*, 2b* vorgesehen, die in der Objektebene einen Stereowinkel α einschließen und über Okulare 22'*, 22"* Augen 37'*, 37"* eines Benutzers zugeführt werden. Entlang der Abbildungsstrahlengänge 2a*, 2b* ist zwischen dem Abbildungssystem 26* und der Objektebene 1* eine separate Spiegelfläche 3* angeordnet, die nicht Teil des Abbildungssystems 26* ist. Der Bereich zwischen der Spiegelfläche 3* und der Objektebene 1* ist somit frei von optisch wirksamen Oberflächen und/oder Elementen.

Die Spiegelfläche 3* ist mit einem Antrieb 36* verbunden, der ausgebildet ist, die Spiegelfläche 3* wahlweise um einen Schwenkpunkt P in eine beliebige Richtung zu verschwenken. Der Schwenkpunkt P liegt in der gezeigten Ausführungsform auf einer durch die optischen Linsen 4*, 5* und 6* festgelegten optischen Achse K des ersten Teilsystems T1*. In Figur 3 liegt der Schwenkpunkt P in der Mitte der Spiegelfläche 3*.

Weiter weist das zweite Teilsystem T2* des in Figur 3 gezeigten Abbildungssystems 26* für jeden Abbildungsstrahlengang 2a*, 2b* eine Kompensationseinrichtung 27'*, 27"* in Form einer einstellbaren Prisma-Anordnung auf.

Antriebe (nicht eigens gezeigt) der Kompensationseinrichtungen 27'*, 27"* sowie der Antrieb 3* der Spiegelfläche 3* sind über Datenleitungen mit einer Steuerung 28 verbunden, die ihrerseits über eine Datenleitung mit einer Benutzerschnittstelle 29 (hier beispielhaft aber nicht beschränkend durch einen Joystick dargestellt) verbunden ist.

In Abhängigkeit von einem über die Benutzerschnittstelle 29 empfangenen Befehl steuert die Steuerung 28 den Antrieb 36* an, um ein (in Figur 3 nicht gezeigtes) Abbildungsfeld des Abbildungssystems 26* in der Objektebene 1 in eine beliebige Richtung zu verlagern. Gleichzeitig steuert die Steuerung 28 die Antriebe der Kompensationseinrichtungen 27'*, 27"* automatisch so, dass die Kompensationseinrichtungen 27'*, 27"* eine durch ein Verschwenken der Spiegelfläche 3* hervorgerufene Bilddrehung automatisch kompensieren.

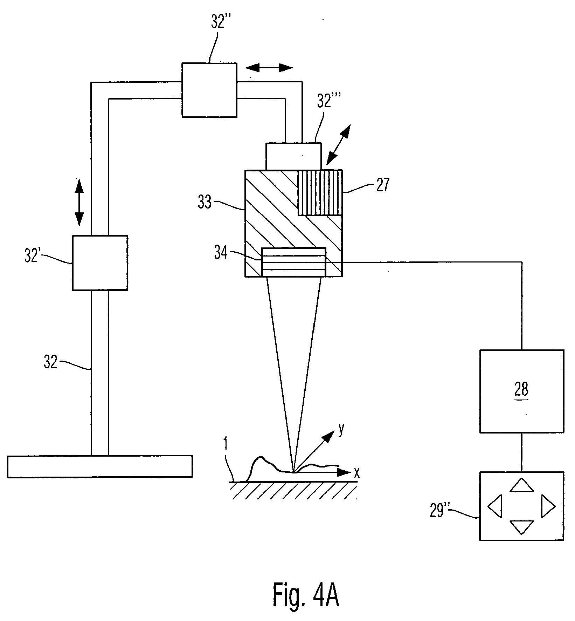

In Figur 4A ist schematisch eine Seitenansicht des Aufbaus des Mikroskopiesystems gemäß der zweiten Ausführungsform der vorliegenden Erfindung gezeigt.

Wie ersichtlich, kann das Mikroskopiesystem weiter ein Stativ 32 umfassen, welches ein Operationsmikroskop 33 trägt. Das Operationsmikroskop 33 umfasst das Abbildungssystem 26, die Kompensationseinrichtung 27 und die Versatzeinrichtung 34 mit jeweils dem in der zweiten Ausführungsform beschriebenen Aufbau. Über Antriebe 32', 32", 32'" ermöglicht das Stativ 32 eine translatorische und rotatorische Verlagerung des Operationsmikroskops 33 relativ zur Objektebene 1.

Zur Eingabe eines gewünschten Versatzes des Abbildungsfeldes relativ zur Objektebene weist die Steuerung 28 in Figur 4A eine Tastatur 29'' auf.

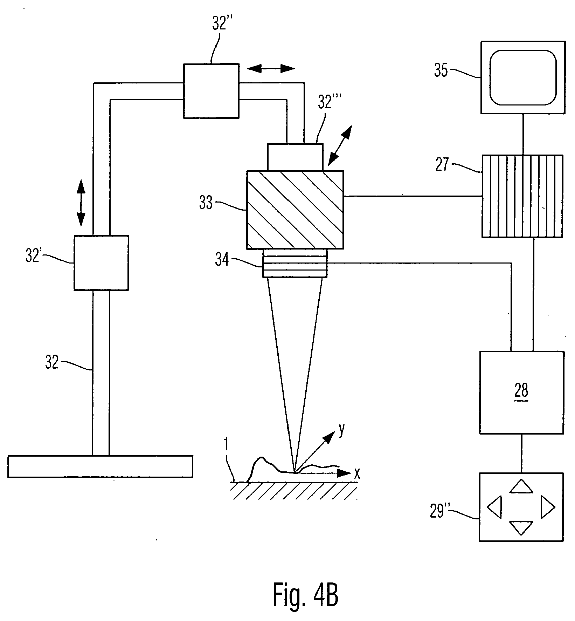

In Figur 4B ist schematisch eine Seitenansicht eines Aufbaus eines Mikroskopiesystems gemäß einer vierten Ausführungsform der vorliegenden Erfindung gezeigt.

Dabei unterscheidet sich die in Figur 4B gezeigte vierte Ausführungsform von der in Figur 4A gezeigten Ausführungsform insbesondere dadurch, dass die Kompensationseinrichtung 27 und die Versatzeinrichtung 34 nicht von dem Operationsmikroskop 33 umfasst werden, sondern separate, mit dem Operationsmikroskop 33 verbindbare Module sind. In Figur 4B wird die Kompensationseinrichtung 27 von einem Grafikprozessor gebildet, der ein von dem Operationsmikroskop 33 empfangendes und in Abhängigkeit von der Steuerung 28 gedrehtes und hinsichtlich seiner Stereobasis korrigiertes Bild über einen Monitor 35 ausgibt.

Auch wenn in den vorstehend beschriebenen Ausführungsformen nur wahlweise ein bzw. zwei verschwenkbare Spiegelflächen vorgesehen sind, um ein Abbildungsfeld des jeweiligen Abbildungssystems in der Objektebene zu verlagern, ist die vorliegende Erfindung hierauf nicht beschränkt. Vielmehr kann eine beliebige Anzahl von verschwenkbaren Spiegelflächen vorgesehen sein, um das Abbildungsfeld des Abbildungssystems in der Objektebene zu verlagern. Weiter kann das Verschenken der Spiegelflächen beispielsweise wahlweise dadurch erfolgen, dass die jeweilige Spiegelfläche um mehr als eine Schwenkachse verschwenkt wird, oder dadurch, dass die jeweilige Spiegelfläche um eine Drehachse rotiert wird, die mit einer Normalen zur Spiegelfläche einen von Null verschiedenen Winkel einschließt. Diese Drehachse kann mit einer auf die jeweilige Spiegelfläche einfallenden optischen Achse zusammenfallen. Durch eine derartige Rotation der Spiegelfläche wird gleichzeitig ein Verschwenken der Spiegelfläche relativ zu der auf die Spiegelfläche einfallenden optischen Achse bewirkt, was einen Versatz des Abbildungsfeldes in der Objektebene zur Folge hat.

Zusammenfassend stellt die vorliegende Erfindung ein Mikroskopiesystem bereit, welches durch das Verschwenken wenigstens einer Spiegelfläche eine Verschiebung eines Abbildungsfeldes eines Abbildungssystems eines Mikroskopiesystems in einer Objektebene auf besonders einfache, zuverlässige und vibrationsfreie Weise ermöglicht. Hierdurch ist es mittels des erfindungsgemäßen Mikroskopiesystems auf besonders bequeme Weise möglich, Bewegungen (und insbesondere auch periodische Bewegungen) eines betrachteten Objektes durch entsprechende Verlagerung des Abbildungsfeldes auszugleichen und so ein betrachtetes Objekt in dem Abbildungsfeld beizubehalten. Der Grund ist, dass lediglich eine Spiegelfläche verschwenkt werden muss und somit auf eine Verlagerung des gesamten Abbildungssystems des Mikroskopiesystems verzichtet werden kann. Die Verlagerung kann durch eine Steuerung kontrolliert werden, welche einen gewünschten Versatz automatisch oder anhand einer Benutzereingabe ermittelt. Zudem kann eine durch ein Verschwenken der wenigstens einen Spiegelfläche ggf. hervorgerufene Rotation der durch das Abbildungssystem erstellten Abbildung eines Objektes automatisch mittels einer Kompensationsreinrichtung korrigiert werden. Hierfür steuert die Steuerung die Kompensationseinrichtung in Abhängigkeit von einem Maß und einer Richtung des Verschwenkens der wenigstens einen Spiegelfläche.

Ein derartiges Mikroskopiesystem eignet sich insbesondere zur Verwendung als Operationsmikroskop.

## Patentansprüche

1. Verfahren, in einer Objektebene (1) ein nichtpunktförmiges Abbildungsfeld (F) zu versetzen, welches Abbildungsfeld (F) durch wenigstens ein Paar von Abbildungsstrahlengängen (2a, 2b, 2c, 2d) eines Abbildungssystems (26) eines Mikroskopiesystems abgebildet wird, wobei das wenigstens eine Paar von Abbildungsstrahlengängen (2a, 2b, 2c, 2d) in der Objektebene (1) einen Stereowinkel (α) einschließt, wobei das Mikroskopiesystem eine Versatzeinrichtung mit einer entlang des wenigstens einen Paars von Abbildungsstrahlengängen angeordneten ersten Spiegelfläche (3) zur Ablenkung des wenigstens einen Paars von Abbildungsstrahlengängen (2a, 2b, 2c, 2d) aufweist, welche erste Spiegelfläche (3) verschwenkbar ist;
wobei das Verfahren umfasst:
Erfassen eines bei ortsfestem Abbildungssystem von einer ersten Position in der Objektebene zu einer von der ersten Position verschiedenen zweiten Position in der Objektebene zu bewirkenden Versatzes des Abbildungsfelds (F); und
Verschwenken der ersten Spiegelfläche (3) in Abhängigkeit von dem erfassten zu bewirkenden Versatz, um so das Abbildungsfeld (F) des Abbildungssystems (26) in der Objektebene (1) in eine erste Richtung zu versetzen;
wobei die Versatzeinrichtung weiter eine entlang des wenigstens einen Paars von Abbildungsstrahlengängen (2a, 2b, 2c, 2d) angeordnete zweite Spiegelfläche (9) zur Ablenkung des wenigstens einen Paars von Abbildungsstrahlengängen (2a, 2b, 2c, 2d) aufweist,
wobei die zweite Spiegelfläche (9) verschwenkbar ist, und
wobei die erste Spiegelfläche (3) um eine erste Schwenkachse (A) und die zweite Spiegelfläche (9) um eine zweite Schwenkachse (B) schwenkbar ist, wobei die zweite Schwenkachse (B) von der ersten Schwenkachse (A) verschieden ist, rund
wobei die erste Schwenkachse (A) mit einer ersten Ablenkungsebene, die von einer auf die erste Spiegelfläche (3) einfallenden und einer von der ersten Spiegelfläche (3) ausfallenden optischen Achse (K; K*) eines Abbildungsstrahlengangs des wenigstens einen Paars von Abbildungsstrahlengängen (2a, 2b, 2c, 2d) aufgespannt ist, einen Winkel von im wesentlichen 90° einschließt, und wobei die zweite Schwenkachse (B) mit einer zweiten Ablenkungsebene, die von der auf die zweite Spiegelfläche (9) einfallenden und einer von der zweiten Spiegelfläche (9) ausfallenden optischen Achse (K; K*) eines Abbildungsstrahlengangs des wenigstens einen Paars von Abbildungsstrahlengängen (2a, 2b, 2c, 2d) aufgespannt ist, einen Winkel von im wesentlichen 90° einschließt und wobei die zweite Schwenkachse (B) zu der ersten Ablenkungsebene im wesentlichen parallel angeordnet ist; und das Verfahren weiter umfasst:
Verschwenken der zweiten Spiegelfläche (9) in Abhängigkeit von dem erfassten zu bewirkenden Versatz, um so das Abbildungsfeld (F) des Abbildungssystems (26) in der Objektebene (1) in eine von der ersten Richtung verschiedenen zweiten Richtung zu versetzen.

2. Verfahren nach Anspruch 1, weiter umfassend:
Rotation der durch das Abbildungssystem erzeugten Abbildung des Abbildungsfelds (F) in Abhängigkeit von einer Verschwenkung der Spiegelfläche (3, 9).

3. Verfahren nach Anspruch 1 oder 2, weiter umfassend:
Detektieren der Position eines Markers in der durch das Abbildungssystem erzeugten Abbildung des Abbildungsfelds (F) und Verschwenken der ersten und zweiten Spiegelfläche (3, 9) in Abhängigkeit von der detektierten Position des Markers so, dass die Position des Markers in der Abbildung im wesentlichen konstant bleibt.

4. Verfahren nach einem der Ansprüche 1 bis 3,
wobei das Mikroskopiesystem weiter ein Beleuchtungssystem (23, 30) mit einem Beleuchtungsstrahlengang (24) zur Beleuchtung der Objektebene (1) umfasst, wobei entlang des Beleuchtungsstrahlengangs (24) wenigstens ein Beleuchtungsspiegel (38) angeordnet ist; und
wobei das Verfahren umfasst:
Verschwenken des Beleuchtungsspiegels (38) in Abhängigkeit von dem erfassten zu bewirkenden Versatz.

5. Mikroskopiesystem zur Abbildung eines in einer Objektebene (1) des Mikroskopiesystems anordenbaren Objekts, wobei das Mikroskopiesystem umfasst:
ein Abbildungssystem (26), welches wenigstens ein Paar von Abbildungsstrahlengängen (2a, 2b, 2c, 2d) zur Abbildung eines nicht-punktförmigen Abbildungsfeldes (F) der Objektebene (1) bereitstellt, welches wenigstens ein Paar von Abbildungsstrahlengängen in der Objektebene (1) einen Stereowinkel (α) einschließt;
eine Versatzeinrichtung, welche ausgebildet ist, das Abbildungsfeld (F) des Abbildungssystems (26) in der Objektebene (1) zu versetzen; und
eine Steuerung (28), welche ausgebildet ist, die Versatzeinrichtung zu steuern;
wobei die Versatzeinrichtung eine entlang des wenigstens einen Paars von Abbildungsstrahlengängen (2a, 2b, 2c, 2d) angeordnete erste Spiegelfläche (3) zur Ablenkung des wenigstens einen Paars von Abbildungsstrahlengängen (2a, 2b, 2c, 2d) aufweist, welche erste Spiegelfläche (3) verschwenkbar ist; wobei die Steuerung (28) ausgebildet ist, einen bei ortsfestem Abbildungssystem zu bewirkenden Versatz des Abbildungsfelds (F) von einer ersten Position in der Objektebene zu einer von der ersten Position verschiedenen zweiten Position in der Objektebene zu erfassen und die Versatzeinrichtung entsprechend zu steuern; und
wobei die erste Spiegelfläche (3) in Abhängigkeit von dem durch die Steuerung erfassten zu bewirkenden Versatz verschwenkbar ist;
**dadurch gekennzeichnet, dass**
die Versatzeinrichtung weiter eine entlang des wenigstens einen Paars von Abbildungsstrahlengängen (2a, 2b, 2c, 2d) angeordnete zweite Spiegelfläche (9) zur Ablenkung des wenigstens einen Paars von Abbildungsstrahlengängen (2a, 2b, 2c, 2d) aufweist, wobei die zweite Spiegelfläche (9) in Abhängigkeit von dem durch die Steuerung (28) ermittelten Versatz verschwenkbar ist; und
die erste Spiegelfläche (3) um eine erste Schwenkachse (A) und die zweite Spiegelfläche (9) um eine zweite Schwenkachse (B) schwenkbar ist, wobei die zweite Schwenkachse (B) von der ersten Schwenkachse (A) verschieden ist;
und
wobei die erste Schwenkachse (A) mit einer ersten Ablenkungsebene, die von einer auf die erste Spiegelfläche (3) einfallenden und einer von der ersten Spiegelfläche (3) ausfallenden optischen Achse (K; K*) eines Abbildungsstrahlengangs des wenigstens einen Paars von Abbildungsstrahlengängen (2a, 2b, 2c, 2d) aufgespannt ist, einen Winkel von im wesentlichen 90° einschließt; und
wobei die zweite Schwenkachse (B) mit einer zweiten Ablenkungsebene, die von der auf die zweite Spiegelfläche (9) einfallenden und einer von der zweiten Spiegelfläche (9) ausfallenden optischen Achse (K; K*) eines Abbildungsstrahlengangs des wenigstens einen Paars von Abbildungsstrahlengängen (2a, 2b, 2c, 2d) aufgespannt ist, einen Winkel von im wesentlichen 90° einschließt und wobei die zweite Schwenkachse (B) zu der ersten Ablenkungsebene im wesentlichen parallel angeordnet ist.

6. Mikroskopiesystem nach Anspruch 5,
wobei das Mikroskopiesystem weiter eine Kompensationseinrichtung (27; 27', 27", 27*; 27'*, 27"*) umfasst, welche eine Rotation der durch das Abbildungssystem erzeugten Abbildung des Abbildungsfelds (F) bewirkt; und
wobei die Steuerung (28) die Kompensationseinrichtung (27; 27', 27", 27*; 27'*, 27"*) in Abhängigkeit von einer Verschwenkung der ersten und/oder zweiten Spiegelfläche (3, 9) steuert;
wobei das Abbildungssystem (26) wenigstens eine in wenigstens einem Abbildungsstrahlengang des wenigstens einen Paars von Abbildungsstrahlengängen (2a, 2b, 2c, 2d) angeordnete Kamera (31"', 31"") zur Erzeugung von Bilddaten aufweist; und
wobei die Kompensationseinrichtung (27; 27*) mit der wenigstens einen Kamera (31"', 31"") verbunden ist und durch elektronische Bildverarbeitung eine Rotation der von der wenigstens einen Kamera (31"', 31"") erzeugten Bilddaten bewirkt.

7. Mikroskopiesystem nach einem der Ansprüche 5 bis 6,
wobei das Abbildungssystem (26) wenigstens eine in wenigstens einem Abbildungsstrahlengang des wenigstens einen Paars von Abbildungsstrahlengängen (2a, 2b, 2c, 2d) angeordnete Kamera (31"', 31"") zur Erzeugung von Bilddaten aufweist; und
wobei die Steuerung (28) mit der wenigstens einen Kamera (31"', 31"") verbunden und weiter ausgebildet ist,
in den Bilddaten die Position eines Markers zu detektieren und die Versatzeinrichtung in Abhängigkeit von der detektierten Position des Markers zu steuern.

8. Mikroskopiesystem nach Anspruch 7, wobei die Steuerung (28) die Versatzeinrichtung automatisch so steuert, dass die Position des Markers in den Bilddaten im wesentlichen konstant bleibt.

9. Mikroskopiesystem nach einem der Ansprüche 5 bis 8,
wobei die Steuerung (28) eine Benutzerschnittstelle (29, 29', 29") aufweist, und den Versatz des Abbildungsfelds (F) in der Objektebene (1) in Abhängigkeit von einem über die Benutzerschnittstelle (29, 29', 29'') empfangenen Steuerbefehl ermittelt, wobei die Benutzerschnittstelle (29, 29', 29") ausgebildet ist, um Steuerbefehle von einem Benutzer in Form von Sprache und/oder einer Augenbewegung und/oder einer Fußbewegung und/oder einer Kopfbewegung und/oder einer Handbewegung des Benutzers zu empfangen und an die Steuerung (28) auszugeben.

10. Mikroskopiesystem nach einem der Ansprüche 5 bis 9,
wobei das Abbildungssystem (26) eine Mehrzahl von optischen Linsen (4-8, 11, 13, 14, 16'-21', 16"-21", 16"'-21"', 16""-21"") umfasst; und
wobei zwischen der ersten und der zweiten Spiegelfläche (3, 9) wenigstens eine optisch Linse (4-8) des Abbildungssystems (26) angeordnet ist.

11. Mikroskopiesystem nach einem der Ansprüche 5 bis 10,
wobei das Abbildungssystem (26) eine dritte Spiegelfläche (10) und eine vierte Spiegelfläche (12) zur Ablenkung des wenigstens einen Abbildungsstrahlengangs (2a, 2b, 2c, 2d) aufweist; und
wobei das wenigstens eine Paar von Abbildungsstrahlengängen (2a, 2b, 2c, 2d) nacheinander an der ersten Spiegelfläche (3), der zweiten Spiegelfläche (9), der dritten Spiegelfläche (10) und der vierten Spiegelfläche (12) reflektiert ist.

12. Mikroskopiesystem nach Anspruch 11, wobei die erste Spiegelfläche (3) und die vierte Spiegelfläche (12) relativ zueinander einen Winkel von zwischen 60° und 120° und bevorzugt zwischen 80° und 100° einschließen, sowie die zweite Spiegelfläche (9) und die dritte Spiegelfläche (10) relativ zueinander einen Winkel von zwischen 60° und 120° und bevorzugt zwischen 80° und 100° einschließen.

13. Mikroskopiesystem nach Anspruch 12, wobei die dritte Spiegelfläche (10) und die vierte Spiegelfläche (9) relativ zueinander einen Winkel von im wesentlichen 60° einschließen.

14. Mikroskopiesystem nach einem der Ansprüche 5 bis 13,
wobei das Mikroskopiesystem weiter einen zweiten Antrieb (37) aufweist, welcher die zweite Spiegelfläche (9) wahlweise um die zweite Schwenkachse (B) verschwenkt, wobei das Mikroskopiesystem weiter einen ersten Antrieb (36) aufweist, welcher die erste Spiegelfläche (3) wahlweise um die erste Schwenkachse (A) verschwenkt.

15. Mikroskopiesystem nach einem der Ansprüche 5 bis 14,
wobei die erste Spiegelfläche (3) zwischen der Objektebene (1) und einer entlang des wenigstens einen Paars von Abbildungsstrahlengängen (2a, 2b, 2c, 2d) ersten optisch wirksamen Oberfläche des Abbildungssystems (26) angeordnet ist.

16. Mikroskopiesystem nach einem der Ansprüche 5 bis 15,
wobei das Abbildungssystem (26) ein erstes Teilsystem (T1; T1*) aufweist, das eine Mehrzahl von Linsen (4-8, 11, 13, 14) umfasst, welche entlang einer gemeinsamen optischen Achse (K; K*) angeordnet sind und von beiden Abbildungsstrahlengängen (2a, 2b, 2c, 2d) des wenigstens einen Paars von Abbildungsstrahlengängen (2a, 2b, 2c, 2d) gemeinsam durchsetzt sind, wobei die erste und/oder zweite Spiegelfläche (3, 9) entlang der optischen Achse (K; K*) des ersten Teilsystems (T1; T1*) zwischen optischen Linsen (4-8, 11, 13, 14) des ersten Teilsystems (T1; T1*) angeordnet sind.

17. Mikroskopiesystem nach einem der Ansprüche 5 bis 16,
wobei das Mikroskopiesystem weiter ein Beleuchtungssystem (23, 30) mit einem Beleuchtungsstrahlengang (24) zur Beleuchtung der Objektebene (1) umfasst;
wobei die erste und/oder zweite Spiegelfläche (3, 9) entlang des Beleuchtungsstrahlengangs angeordnet ist; und
wobei der Beleuchtungsstrahlengang wenigstens durch die erste und/oder zweite Spiegelfläche (3, 9) abgelenkt ist.

18. Mikroskopiesystem nach einem der Ansprüche 5 bis 17,
wobei das Mikroskopiesystem weiter ein Beleuchtungssystem (23, 30) mit einem Beleuchtungsstrahlengang (24) zur Beleuchtung der Objektebene (1) umfasst; und
wobei entlang des Beleuchtungsstrahlengangs (24) wenigstens ein Beleuchtungsspiegel (38) angeordnet ist, welcher in Abhängigkeit von dem durch die Steuerung (28) ermittelten Versatz verschwenkbar ist.

19. Mikroskopiesystem nach einem der Ansprüche 5 bis 18,
wobei das Mikroskopiesystem weiter ein Stativ (32) umfasst, welches das Abbildungssystem (26) trägt und wenigstens eine Verstellvorrichtung (32', 32", 32"') zur translatorischen Verlagerung des Abbildungssystems aufweist.

## Claims

1. Method for offsetting a non-punctiform imaging field (F) in an object plane (1), which imaging field (F) is imaged by at least one pair of imaging beam paths (2a, 2b, 2c, 2d) of an imaging system (26) of a microscopy system, wherein the at least one pair of imaging beam paths (2a, 2b, 2c, 2d) includes a stereo angle (□) in the object plane (1), wherein the microscopy system has an offset apparatus with a first mirror face (3), arranged along the at least one pair of imaging beam paths, for deflecting the at least one pair of imaging beam paths (2a, 2b, 2c, 2d), which first mirror face (3) is swivelable;
wherein the method comprises the following:
acquiring an offset of the imaging field (F) to be brought about in the case of a stationary imaging system from a first position in the object plane to a second position, different from the first position, in the object plane; and swivelling the first mirror face (3) in a manner dependent on the acquired offset to be brought about in order thus to offset the imaging field (F) of the imaging system (26) in a first direction in the object plane (1);
wherein the offset apparatus furthermore has a second mirror face (9), arranged along the at least one pair of imaging beam paths (2a, 2b, 2c, 2d), for deflecting the at least one pair of imaging beam paths (2a, 2b, 2c, 2d), wherein the second mirror face (9) is swivelable, and wherein the first mirror face (3) is swivelable about a first swivel axis (A) and the second mirror face (9) is swivelable about a second swivel axis (B), wherein the second swivel axis (B) differs from the first swivel axis (A) and wherein the first swivel axis (A) includes an angle of substantially 90° with a first deflection plane, which is spanned by an optical axis (K; K*), incident on the first mirror face (3) and reflected by the first mirror face (3), of an imaging beam path of the at least one pair of imaging beam paths (2a, 2b, 2c, 2d) and wherein the second swivel axis (B) includes an angle of substantially 90° with a second deflection plane, which is spanned by the optical axis (K; K*), incident on the second mirror face (9) and reflected by the second mirror face (9), of an imaging beam path of the at least one pair of imaging beam paths (2a, 2b, 2c, 2d), and wherein the second swivel axis (B) is arranged substantially parallel to the first deflection plane; and
the method furthermore comprises:
swivelling the second mirror face (9) in a manner dependent on the acquired offset to be brought about in order thus to offset the imaging field (F) of the imaging system (26) in a second direction, different from the first direction, in the object plane (1).

2. Method according to Claim 1, furthermore comprising:
rotating the image of the imaging field (F) generated by the imaging system in a manner dependent on a swivel of the mirror face (3, 9).

3. Method according to Claim 1 or 2, furthermore comprising:
detecting the position of a mark in the image of the imaging field (F) generated by the imaging system and swivelling the first and second mirror face (3, 9) in a manner dependent on the detected position of the mark in such a way that the position of the mark in the image remains substantially constant.

4. Method according to one of Claims 1 to 3,
wherein the microscopy system furthermore comprises an illumination system (23, 30) with an illumination beam path (24) for illuminating the object plane (1), wherein at least one illumination mirror (38) is arranged along the illumination beam path (24); and
wherein the method comprises the following:
swivelling the illumination mirror (38) in a manner dependent on the acquired offset to be brought about.

5. Microscopy system for imaging an object arrangeable in an object plane (1) of the microscopy system, said microscopy system comprising:
an imaging system (26), which provides at least one pair of imaging beam paths (2a, 2b, 2c, 2d) for imaging a non-punctiform imaging field (F) in the object plane (1), which at least one pair of imaging beam paths includes a stereo angle (□) in the object plane (1);
an offset apparatus embodied to offset the imaging field (F) of the imaging system (26) in the object plane (1); and
a controller (28) embodied to control the offset apparatus;
wherein the offset apparatus has a first mirror face (3), arranged along the at least one pair of imaging beam paths (2a, 2b, 2c, 2d), for deflecting the at least one pair of imaging beam paths (2a, 2b, 2c, 2d), which first mirror face (3) is swivelable;
wherein the controller (28) is embodied to acquire an offset of the imaging field (F) to be brought about in the case of a stationary imaging system from a first position in the object plane to a second position, different from the first position, in the object plane and control the offset apparatus accordingly; and
wherein the first mirror face (3) is swivelable in a manner dependent on the offset to be brought about that was acquired by the controller;
**characterized in that**
the offset apparatus furthermore has a second mirror face (9), arranged along the at least one pair of imaging beam paths (2a, 2b, 2c, 2d), for deflecting the at least one pair of imaging beam paths (2a, 2b, 2c, 2d), wherein the second mirror face (9) is swivelable in a manner dependent on the offset established by the controller (28); and
the first mirror face (3) is swivelable about a first swivel axis (A) and the second mirror face (9) is swivelable about a second swivel axis (B), wherein the second swivel axis (B) differs from the first swivel axis (A); and wherein the first swivel axis (A) includes an angle of substantially 90° with a first deflection plane, which is spanned by an optical axis (K; K*), incident on the first mirror face (3) and reflected by the first mirror face (3), of an imaging beam path of the at least one pair of imaging beam paths (2a, 2b, 2c, 2d); and
wherein the second swivel axis (B) includes an angle of substantially 90° with a second deflection plane, which is spanned by the optical axis (K; K*), incident on the second mirror face (9) and reflected by the second mirror face (9), of an imaging beam path of the at least one pair of imaging beam paths (2a, 2b, 2c, 2d), and wherein the second swivel axis (B) is arranged substantially parallel to the first deflection plane.

6. Microscopy system according to Claim 5,
wherein the microscopy system furthermore comprises a compensation apparatus (27; 27', 27", 27*; 27'*, 27"*), which brings about a rotation of the image of the imaging field (F) produced by the imaging system; and wherein the controller (28) controls the compensation apparatus (27; 27', 27", 27*; 27'*, 27"*) in a manner dependent on a swivelling of the first and/or second mirror face (3, 9);
wherein the imaging system (26) has at least one camera (31"', 31""), arranged in at least one imaging beam path of the at least one pair of imaging beam paths (2a, 2b, 2c, 2d), for producing image data; and
wherein the compensation apparatus (27; 27*) is connected to the at least one camera (31"', 31"") and it brings about a rotation of the image data produced by the at least one camera (31"', 31"") by way of electronic image processing.

7. Microscopy system according to either of Claims 5 and 6,
wherein the imaging system (26) has at least one camera (31"', 31""), arranged in at least one imaging beam path of the at least one pair of imaging beam paths (2a, 2b, 2c, 2d), for producing image data; and
wherein the controller (28) is connected to the at least one camera (31"', 31"") and furthermore embodied to detect the position of a mark in the image data and to control the offset apparatus in a manner dependent on the detected position of the mark.

8. Microscopy system according to Claim 7, wherein the controller (28) controls the offset apparatus automatically in such a way that the position of the mark remains substantially constant in the image data.

9. Microscopy system according to one of Claims 5 to 8, wherein the controller (28) has a user interface (29, 29', 29") and establishes the offset of the imaging field (F) in the object plane (1) in a manner dependent on a control command received by way of the user interface (29, 29', 29"), wherein the user interface (29, 29', 29") is embodied to receive control commands from a user in the form of speech and/or an eye movement and/or a foot movement and/or a head movement and/or a hand movement of the user and to output said control commands to the controller (28).

10. Microscopy system according to one of Claims 5 to 9,
wherein the imaging system (26) comprises a plurality of optical lenses (4-8, 11, 13, 14, 16'-21', 16"-21", 16"'-21"', 16"'-21"'); and
wherein at least one optical lens (4-8) of the imaging system (26) is arranged between the first and the second mirror face (3, 9).

11. Microscopy system according to one of Claims 5 to 10,
wherein the imaging system (26) has a third mirror face (10) and a fourth mirror face (12) for deflecting the at least one imaging beam path (2a, 2b, 2c, 2d); and
wherein the at least one pair of imaging beam paths (2a, 2b, 2c, 2d) is successively reflected at the first mirror face (3), the second mirror face (9), the third mirror face (10) and the fourth mirror face (12).

12. Microscopy system according to Claim 11, wherein the first mirror face (3) and the fourth mirror face (12) include an angle of between 60° and 120° and of preferably between 80° and 100° relative to one another and the second mirror face (9) and the third mirror face (10) include an angle of between 60° and 120° and of preferably between 80° and 100° relative to one another.

13. Microscopy system according to Claim 12, wherein the third mirror face (10) and the fourth mirror face (9) include an angle of substantially 60° relative to one another.

14. Microscopy system according to one of Claims 5 to 13, wherein the microscopy system furthermore has a second drive (37), which optionally swivels the second mirror face (9) about the second swivel axis (B), wherein the microscopy system furthermore has a first drive (36), which optionally swivels the first mirror face (3) about the first swivel axis (A).

15. Microscopy system according to one of Claims 5 to 14, wherein the first mirror face (3) is arranged between the object plane (1) and a first optically effective surface of the imaging system (26) along the at least one pair of imaging beam paths (2a, 2b, 2c, 2d).

16. Microscopy system according to one of Claims 5 to 15,
wherein the imaging system (26) has a first subsystem (T1; T1*) comprising a plurality of lenses (4-8, 11, 13, 14), which are arranged along a common optical axis (K; K*) and which are passed-through together by both imaging beam paths (2a, 2b, 2c, 2d) of the at least one pair of imaging beam paths (2a, 2b, 2c, 2d), wherein the first and/or second mirror face (3, 9) is/are arranged between optical lenses (4-8, 11, 13, 14) of the first subsystem (T1; T1*) along the optical axis (K; K*) of the first subsystem (T1; T1*).

17. Microscopy system according to one of Claims 5 to 16,
wherein the microscopy system furthermore comprises an illumination system (23, 30) with an illumination beam path (24) for illuminating the object plane (1);
wherein the first and/or second mirror face (3, 9) is/are arranged along the illumination beam path; and
wherein the illumination beam path is deflected by at least the first and/or second mirror face (3, 9).

18. Microscopy system according to one of Claims 5 to 17,
wherein the microscopy system furthermore comprises an illumination system (23, 30) with an illumination beam path (24) for illuminating the object plane (1); and
wherein at least one illumination mirror (38), which is swivelable in a manner dependent on the offset established by the controller (28), is arranged along the illumination beam path (24).

19. Microscopy system according to one of Claims 5 to 18, wherein the microscopy system furthermore comprises a stand (32), which carries the imaging system (26) and has at least one adjustment device (32', 32", 32"') for translational displacement of the imaging system.

## Revendications

1. Procédé pour déplacer dans un plan d'objet (1) un champ non ponctuel (F) de formation d'image, l'image du champ (F) de formation d'image étant formée par au moins une paire de faisceaux de rayons (2a, 2b, 2c, 2d) de formation d'image d'un système (26) de formation d'image d'un système de microscopie,
la ou les paires de faisceaux de rayons (2a, 2b, 2c, 2d) de formation d'image formant un angle stéréoscopique (α) dans le plan d'objet (1),
le système de microscopie présentant un dispositif de déplacement présentant un décalage qui présente une première surface réfléchissante (3) disposée le long de la ou des paires de faisceaux de rayons de formation d'image et déviant la ou les paires de faisceaux de rayons (2a, 2b, 2c, 2d) de formation d'image, la première surface réfléchissante (3) pouvant pivoter,
le procédé comportant les étapes qui consistent à :
saisie du décalage du champ (F) de formation d'image qu'il faut effectuer, le système de formation d'image étant fixe, depuis une première position située dans le plan d'objet jusque dans une deuxième position située dans le plan d'objet et différente de la première position,
pivotement de la première surface réfléchissante (3) en fonction du décalage à réaliser qui a été saisi, pour ainsi décaler le champ (F) de formation d'image du système (26) de formation d'image dans une première direction dans le plan d'objet (1),
le dispositif de décalage présentant en outre une deuxième surface réfléchissante (9) disposée le long de la ou des paires de faisceaux de rayons (2a, 2b, 2c, 2d) de formation d'image pour dévier la ou les paires de faisceaux de rayons (2a, 2b, 2c, 2d) de formation d'image,
la deuxième surface réfléchissante (9) pouvant pivoter,
la première surface réfléchissante (3) pouvant pivoter autour d'un premier axe de pivotement (A) et la deuxième surface réfléchissante (9) autour d'un deuxième axe de pivotement (B),
le deuxième axe de pivotement (B) étant différent du premier axe de pivotement (A),
le premier axe de pivotement (A) formant un angle d'essentiellement 90° avec un premier plan de déviation sous-tendu par un axe optique (K) incident sur la première surface réfléchissante (3) et un axe optique (K*) sortant de la surface réfléchissante (3) d'un faisceau de rayons de formation d'image de la ou des paires de faisceaux de rayons (2a, 2b, 2c, 2d) de formation d'image,
le deuxième axe optique (B) formant un angle d'essentiellement 90° avec un deuxième plan de déviation qui est sous-tendu par un axe optique (K) incident sur la deuxième surface réfléchissante (9) et un axe optique (K*), sortant de la deuxième surface réfléchissante (9), d'un faisceau de rayons de formation d'image de la ou des paires de faisceaux de rayons (2a, 2b, 2c, 2d) de formation d'image,
le deuxième axe optique (B) étant essentiellement parallèle au premier plan de déviation,
le procédé comportant en outre l'étape qui consiste à :
faire pivoter la deuxième surface réfléchissante (9) en fonction du décalage à réaliser qui a été saisi, pour ainsi décaler le champ (F) de formation d'image du système (26) de formation d'image dans une deuxième direction, différente de la première direction, dans le plan d'objet (1).

2. Procédé selon la revendication 1, comprenant en outre l'étape de rotation de l'image du champ (F) de formation d'image formée par le système de formation d'image en fonction d'un pivotement de la surface réfléchissante (3, 9).

3. Procédé selon les revendications 1 ou 2, comprenant en outre la détection de la position d'un repère située dans l'image du champ (F) de formation d'image formée par le système de formation d'image et pivotement de la première et de la deuxième surface réfléchissante (3, 9) en fonction de la position du marqueur qui a été détectée, de telle sorte que la position du marqueur reste essentiellement constante dans l'image formée.

4. Procédé selon l'une des revendications 1 à 3, dans lequel le système de microscopie comporte en outre un système d'éclairage (23, 30) doté d'un faisceau d'éclairage (24) qui éclaire le plan d'objet (1), au moins un miroir d'éclairage (38) étant disposé sur le faisceau d'éclairage (24) et le procédé comportant en outre le pivotement du miroir d'éclairage (38) en fonction du décalage à réaliser qui a été saisi.

5. Système de microscopie destiné à former l'image d'un objet qui peut être disposé dans un plan d'image (1) du système de microscopie, le système de microscopie comprenant :
un système (26) de formation d'image qui forme au moins une paire de faisceaux de rayons (2a, 2b, 2c, 2d) de formation d'image formant l'image d'un champ non ponctuel (F) de formation d'image du plan d'objet (1), la ou les paires de faisceaux de rayons de formation d'image formant un angle stéréoscopique (α) dans le plan d'objet (1),
un dispositif de décalage configuré pour décaler le champ (F) de formation d'image du système (26) de formation d'image dans le plan d'objet (1) et
une commande (28) conçue pour commander le dispositif de décalage,
le dispositif de décalage présentant une première surface réfléchissante (3) disposée le long de la ou des paires de faisceaux de rayons (2a, 2b, 2c, 2d) de formation d'image pour dévier la ou les paires de faisceaux de rayons (2a, 2b, 2c, 2d) de formation d'image, la première surface réfléchissante (3) pouvant pivoter,
la commande (28) étant configurée pour saisir un décalage du champ (F) de formation d'image à réaliser pendant que le système de formation d'image est immobile depuis une première position située dans le plan d'objet jusque dans une deuxième position située dans le plan d'objet et différente de la première position, et pour commander de manière appropriée le dispositif de décalage,
la première surface réfléchissante (3) pouvant pivoter en fonction du décalage à réaliser et saisi par la commande,
**caractérisé en ce que**
le dispositif de décalage présente en outre une deuxième surface réfléchissante (9) disposée le long de la ou des paires de faisceaux de rayons (2a, 2b, 2c, 2d) de formation d'image pour dévier la ou les paires de faisceaux de rayons (2a, 2b, 2c, 2d) de formation d'image,
**en ce que** la deuxième surface réfléchissante (9) peut pivoter en fonction du décalage déterminé par la commande (28),
**en ce que** la première surface réfléchissante (3) peut pivoter autour d'un premier axe de pivotement (A) et la deuxième surface réfléchissante (9) autour d'un deuxième axe de pivotement (B),
**en ce que** le deuxième axe de pivotement (B) est différent du premier axe de pivotement (A),
**en ce que** le premier axe de pivotement (A) forme un angle d'essentiellement 90° avec un premier plan de déviation sous-tendu par un axe optique (K) incident sur la première surface réfléchissante (3) et un axe optique (K*) sortant de la surface réfléchissante (3) d'un faisceau de rayons de formation d'image de la ou des paires de faisceaux de rayons (2a, 2b, 2c, 2d) de formation d'image,
**en ce que** le deuxième axe optique (B) forme un angle d'essentiellement 90° avec un deuxième plan de déviation qui est sous-tendu par un axe optique (K) incident sur la deuxième surface réfléchissante (9) et un axe optique (K*), sortant de la deuxième surface réfléchissante (9), d'un faisceau de rayons de formation d'image de la ou des paires de faisceaux de rayons (2a, 2b, 2c, 2d) de formation d'image et
**en ce que** le deuxième axe optique (B) est essentiellement parallèle au premier plan de déviation.

6. Système de microscopie selon la revendication 5, dans lequel le système de microscopie comprend en outre un dispositif de compensation (27; 27', 27", 27*; 27'*, 27"*) qui a pour effet une rotation de l'image du champ (F) de formation d'image formée par le système de formation d'image, la commande (28) commandant le dispositif de compensation (27; 27', 27", 27*; 27'*, 27"*) en fonction d'un pivotement de la première et/ou de la deuxième surface réfléchissante (3, 9), le système (26) de formation d'image présentant au moins une caméra (31"', 31"") de formation de données d'image disposée dans au moins un faisceau de rayons de formation d'image de la ou des paires de faisceaux de rayons (2a, 2b, 2c, 2d) de formation d'image et le dispositif de compensation 27; 27*) étant raccordé à la ou aux caméras (31'", 31"") et ayant pour effet par traitement électronique d'image une rotation des données d'image formées par la ou les caméras (31"',31"").

7. Système de microscopie selon l'une des revendications 5 et 6, dans lequel le système (26) de formation d'image présente au moins une caméra (31"', 31"") disposée dans au moins un faisceau de rayons de formation d'image de la ou des paires de faisceaux de rayons (2a, 2b, 2c, 2d) de formation d'image et formant des données d'image et la commande (28) étant raccordée à la ou aux caméras (31"', 31"") et étant en outre configurée pour détecter dans les données d'image la position d'un marqueur et commander le dispositif de décalage en fonction de la position du marqueur qui a été détectée.

8. Système de microscopie selon la revendication 7, dans lequel la commande (28) commande le dispositif de décalage de manière automatique de telle sorte que la position du repère reste essentiellement constante dans les données d'image.

9. Système de microscopie selon l'une des revendications 5 à 8, dans lequel la commande (28) présente une interface d'utilisateur (29, 29', 29") et détermine le décalage du champ (F) de formation d'image dans le plan d'objet (1) en fonction d'un ordre de commande reçu par l'intermédiaire de l'interface d'utilisateur (29, 29', 29"), l'interface d'utilisateur (29, 29', 29") étant configurée pour recevoir les ordres de commande d'un utilisateur sous forme vocale, d'un déplacement oculaire, d'un déplacement du pied, d'un déplacement de la tête et/ou d'un déplacement de la main de l'utilisateur et de les délivrer à la commande (28).

10. Système de microscopie selon l'une des revendications 5 à 9, dans lequel le système (26) de formation d'image comporte plusieurs lentilles optiques (4-8, 11, 13, 14, 16'-21', 16"-21", 16"'-21'", 16""-21"") et au moins une lentille optique (4-8) du système (26) de formation d'image est disposée entre la première et la deuxième surface réfléchissante (3, 9).

11. Système de microscopie selon l'une des revendications 5 à 10, dans lequel le système (26) de formation d'image présente une troisième surface réfléchissante (10) et une quatrième surface réfléchissante (12) qui dévient le ou les faisceaux de rayons (2a, 2b, 2c, 2d) de formation d'image et la ou les paires de faisceaux de rayons (2a, 2b, 2c, 2d) de formation d'image étant réfléchies successivement sur la première surface réfléchissante (3), la deuxième surface réfléchissante (9), la troisième surface réfléchissante (10) et la quatrième surface réfléchissante (12).

12. Système de microscopie selon la revendication 11, dans lequel la première surface réfléchissante (3) et la quatrième surface réfléchissante (12) forment entre elles un angle compris entre 60° et 120° et de préférence entre 80° et 100°, la deuxième surface réfléchissante (9) et la troisième surface réfléchissante (10) formant entre elles un angle compris entre 60° et 120° et de préférence entre 80° et 100°.

13. Système de microscopie selon la revendication 12, dans lequel la troisième surface réfléchissante (10) et la quatrième surface réfléchissante (9) forment entre elles un angle essentiellement de 60°.

14. Système de microscopie selon l'une des revendications 5 à 13, dans lequel le système de microscopie présente en outre un deuxième entraînement (37) qui fait pivoter sélectivement la deuxième surface réfléchissante (9) autour du deuxième axe de pivotement (B), le système de microscopie présentant en outre un premier entraînement (36) qui fait pivoter sélectivement la première surface réfléchissante (3) autour du premier axe de pivotement (A).

15. Système de microscopie selon l'une des revendications 5 à 14, dans lequel la première surface réfléchissante (3) est disposée entre le plan d'objet (1) et une première surface optiquement active du système (26) de formation d'image disposée le long de la ou des paires de faisceaux de rayons (2a, 2b, 2c, 2d) de formation d'image.

16. Système de microscopie selon l'une des revendications 5 à 15, dans lequel le système (26) de formation d'image présente un premier système partiel (T1; T1*) qui comporte plusieurs lentilles (4-8, 11, 13, 14) disposées le long d'un axe optique commun (K; K*) et traversées conjointement par les deux faisceaux de rayons (2a, 2b, 2c, 2d) de formation d'image de la ou des paires de faisceaux de rayons (2a, 2b, 2c, 2d) de formation d'image, la première et/ou la deuxième surface réfléchissante (3, 9) étant disposées le long de l'axe optique (K; K*) du premier système partiel (T1; T1*) entre des lentilles optiques (4-8, 11, 13, 14) du premier système partiel (T1; T1*).

17. Système de microscopie selon l'une des revendications 5 à 16, dans lequel le système de microscopie présente en outre un système d'éclairage (23, 30) doté d'un faisceau d'éclairage (24) qui éclaire le plan d'objet (1), la première et/ou la deuxième surface réfléchissante (3, 9) étant disposées le long du faisceau d'éclairage et le faisceau d'éclairage étant dévié au moins par la première et/ou la deuxième surface réfléchissante (3, 9).

18. Système de microscopie selon l'une des revendications 5 à 17, dans lequel le système de microscopie présente en outre un système d'éclairage (23, 30) doté d'un faisceau d'éclairage (24) qui éclaire le plan d'objet (1) et au moins un miroir d'éclairage (38) qui peut être pivoté en fonction du décalage déterminé par la commande (28) étant disposé le long du faisceau d'éclairage (24).

19. Système de microscopie selon l'une des revendications 5 à 18, dans lequel le système de microscopie comporte en outre un statif (32) qui porte le système (26) de formation d'image et qui présente au moins un ensemble d'ajustement (32', 32", 32"') qui déplace en translation le système de formation d'image.
